# EUROPEAN PATENT SPECIFICATION

(11) **EP 2 405 750 B1**
(45) Date of publication and mention of the grant of the patent: **09.05.2018**
(21) Application number: 10751482.0
(22) Date of filing: 12.03.2010
(51) Int. Cl.: A01N 37/18, A61K 31/16, A61K 45/06, A61P 35/00

(54) **COMBINATION THERAPY WITH THIOCOLCHICINE DERIVATIVES**
KOMBINATIONSTHERAPIE MIT THIOCOLCHICINDERIVATEN
POLYTHÉRAPIE AVEC DES DÉRIVÉS DE THIOCOLCHICINE

(30) Priority: 13.03.2009 US 210074 P
(43) Date of publication of application: 18.01.2012
(73) Proprietor: Abraxis BioScience, LLC, Summit, New Jersey 07901 (US)
(72) Inventor: DESAI, Neil, P., Pacific Palisades CA 90272 (US); TRIEU, Vuong, Agoura Hills CA 91301 (US)
(74) Representative: Weber, Martin
(86) International application number: PCT/US2010/027159
(87) International publication number: WO 2010/105172

(56) References cited:
- US-A1- 2006 263 434
- US-A1- 2007 116 774
- US-A1- 2007 166 388
- US-A1- 2008 280 987

## Description

### RELATED APPLICATIONS

This application claims priority benefit to provisional applications 61/210,074, filed on March 13, 2009.

### TECHNICAL FIELD

The present invention relates to compositions for used in methods for the treatment of proliferative diseases, wherein the compositions comprise a colchicine or thiocolchicine dimer, wherein the method comprises further administration of an anti-VEGF antibody and a taxane.

### BACKGROUND

The failure of a significant number of tumors to respond to drug and/or radiation therapy is a serious problem in the treatment of cancer. In fact, this is one of the main reasons why many of the most prevalent forms of human cancer still resist effective chemotherapeutic intervention, despite certain advances in the field of chemotherapy.

Cancer is now primarily treated with one or a combination of three types of therapies: surgery, radiation, and chemotherapy. Surgery is a traditional approach in which all or part of a tumor is removed from the body. Surgery generally is only effective for treating the earlier stages of cancer. While surgery is sometimes effective in removing tumors located at certain sites, for example, in the breast, colon, and skin, it cannot be used in the treatment of tumors located in other areas, inaccessible to surgeons, nor in the treatment of disseminated neoplastic conditions such as leukemia. For more than 50% of cancer individuals, by the time they are diagnosed they are no longer candidates for effective surgical treatment. Surgical procedures may increase tumor metastases through blood circulation during surgery. Most of cancer individuals do not die from the cancer at the time of diagnosis or surgery, but rather die from the metastasis and the recurrence of the cancer.

Other therapies are also often ineffective. Radiation therapy is only effective for individuals who present with clinically localized disease at early and middle stages of cancer, and is not effective for the late stages of cancer with metastasis. Radiation is generally applied to a defined area of the subject's body which contains abnormal proliferative tissue, in order to maximize the dose absorbed by the abnormal tissue and minimize the dose absorbed by the nearby normal tissue. However, it is difficult (if not impossible) to selectively administer therapeutic radiation to the abnormal tissue. Thus, normal tissue proximate to the abnormal tissue is also exposed to potentially damaging doses of radiation throughout the course of treatment. There are also some treatments that require exposure of the subject's entire body to the radiation, in a procedure called "total body irradiation", or "TBI." The efficacy of radiotherapeutic techniques in destroying abnormal proliferative cells is therefore balanced by associated cytotoxic effects on nearby normal cells. Because of this, radiotherapy techniques have an inherently narrow therapeutic index which results in the inadequate treatment of most tumors. Even the best radiotherapeutic techniques may result in incomplete tumor reduction, tumor recurrence, increasing tumor burden, and induction of radiation resistant tumors.

Chemotherapy involves the disruption of cell replication or cell metabolism. Chemotherapy can be effective, but there are severe side effects, e.g., vomiting, low white blood cells (WBC), loss of hair, loss of weight and other toxic effects. Because of the extremely toxic side effects, many cancer individuals cannot successfully finish a complete chemotherapy regime. Chemotherapy-induced side effects significantly impact the quality of life of the individual and may dramatically influence individual compliance with treatment. Additionally, adverse side effects associated with other agents are generally the major dose-limiting toxicity (DLT) in the administration of these drugs. For example, mucositis is one of the major dose limiting toxicity for several anticancer agents, including the antimetabolite cytotoxic agents 5-FU, methotrexate, and antitumor antibiotics, such as doxorubicin. Many of these chemotherapy-induced side effects if severe may lead to hospitalization, or require treatment with analgesics for the treatment of pain. Some cancer individuals die from the chemotherapy due to poor tolerance to the chemotherapy. The extreme side effects of anticancer drugs are caused by the poor target specificity of such drugs. The drugs circulate through most normal organs of individuals as well as intended target tumors. The poor target specificity that causes side effects also decreases the efficacy of chemotherapy because only a fraction of the drugs is correctly targeted. The efficacy of chemotherapy is further decreased by poor retention of the anti-cancer drugs within the target tumors.

Due to the severity and breadth of neoplasm, tumor and cancer, there is a great need for effective treatments of such diseases or disorders that overcome the shortcomings of surgery, chemotherapy, and radiation treatment.

Paclitaxel has been shown to have significant antineoplastic and anticancer effects in drug-refractory ovarian cancer and has shown excellent antitumor activity in a wide variety of tumor models, and also inhibits angiogenesis when used at very low doses (Grant et al., Int. J. Cancer, 2003). The poor aqueous solubility of paclitaxel, however, presents a problem for human administration. Indeed, the delivery of drugs that are inherently insoluble or poorly soluble in an aqueous medium can be seriously impaired if oral delivery is not effective. Accordingly, currently used paclitaxel formulations (e.g., Taxol®) require a Cremophor® to solubilize the drug. The presence of Cremophor® in this formulation has been linked to severe hypersensitivity reactions in animals (Lorenz et al., Agents Actions 7:63-67 (1987)) and humans (Weiss et al., J. Clin. Oncol. 8:1263-68 (1990)) and consequently requires premedication of individuals with corticosteroids (dexamethasone) and antihistamines. It was also reported that clinically relevant concentrations of the formulation vehicle Cremophor® EL in Taxol® nullify the antiangiogenic activity of paclitaxel, suggesting that this agent or other anticancer drugs formulated in Cremophor® EL may need to be used at much higher doses than anticipated to achieve effective metronomic chemotherapy (Ng et al., Cancer Res., 64:821-824 (2004)). As such, the advantage of the lack of undesirable side effects associated with low-dose paclitaxel regimes vs. conventional MTD chemotherapy may be compromised. See also U.S. Patent Pub. No. 2004/0143004; WO00/64437. It has been found that nanoparticle compositions of a taxane (such as albumin bound paclitaxel (Abraxane®)) have significantly lower toxicities than other taxanes like Taxol® and Taxotere® with significantly improved outcomes in both safety and efficacy.

Anti-angiogenic agents that specifically target angiogenesis have been developed for treating angiogenesis-associated diseases. See, e.g., U.S. Pat. No. 6,919,309; U.S. Pat. App. Pub. No. 2006/0009412; and PCT App. Pub. Nos. WO04/027027 and WO05/117876. In addition, agents that target established vasculatures (so called "Vascular Targeting Agents" or VTAs) have also been developed. These agents are believed to function by selectively destabilizing the microtubule cytoskeleton of endothelial cells, causing a profound alteration in the shape of the cells which ultimately leads to occlusion of the blood vessel and shutdown of blood flow. See, e.g., WO 2005/113532.

Thiocolchicine dimers are hydrophobic compounds that have been previously described. See, e.g., U.S. Pat. No. 6,627,774. These compounds have dual mechanisms of action, i.e., the compounds have both anti-microtubule activities and topoisomerase I inhibitory activities. Raspaglio et al., Biochem. Pharmacol. 2005, 69(1):113-21. Nanoparticle albumin-bound formulations of thiocolchicine dimers *Nab-*5404 and *Nab-*5676 have been developed as cytotoxic chemotherapeutic agents for treating cancer. See, for example, Bernacki et al., Proc. Amer. Assoc. Cancer Res., vol. 46, 2005 #2390 and PCT Pat. App. No. PCT/US2006/006167. It was found that *Nab-*5404*,* when administered intravenously at 24 mg/kg, qd x 5, was capable of inducing complete tumor regressions and cures in A121 ovarian tumor xenograft.

US 2007/166388 A1 teaches combination therapy methods of treating proliferative diseases (such as cancer) comprising a first therapy comprising administering to an individual a taxane in a nanoparticle composition, and a second therapy which may include, for example, radiation, surgery, administration of chemotherapeutic agents (such as an anti-VEGF antibody), or combinations thereof.

US 2006/263434 A1 teaches methods of treating proliferative diseases in an individual comprising administering to the individual: a) a composition comprising nanoparticles comprising a taxane and an albumin, and b) at least one other chemotherapeutic agent, wherein said chemotherapeutic agent is selected from the group consisting of antimetabolites, platinum-based agents, alkylating agents, tyrosine kinase inhibitors, anthracycline antibiotics, vinca alkloids, proteasome inhibitors, macrolides, and topoisomerase inhibitors.

US 2008/280987 A1 teaches methods of inhibiting angiogenesis, as age-related macular degeneration, diabetic retinopathy, rheumatic arthritis, psoriasis and cancer, in an individual by administering a composition (such as protein containing composition) comprising colchicine or thiocolchicine dimer.

US 2007/116774 A1 relates to methods and compositions for the treatment of proliferative diseases comprising the administration of a combination of a taxane and at least one other and other therapeutic agents, as well as other treatment modalities useful in the treatment of proliferative diseases.

Other references include U.S. Pub. No. 2006/0013819; U.S. Pub. No. 2006/0003931; 20060263434, 20070166388, and PCT Application Nos. WO05/117986; WO05/117978; WO05/000900, WO06/089290, WO08/057562, WO08/027055, and WO08/057562.

### BRIEF SUMMARY OF THE INVENTION

The present invention provides a composition comprising a colchicine or thiocolchicine dimer for use in a method of treating a proliferative disease in an individual, wherein the method comprises further administration of an anti-VEGF antibody and a taxane.

In one embodiment, the colchicine or thiocochicine dimer is IDN-5404.

In one embodiment, the colchicine or thiocolchicine dimer is in the form of nanoparticles.

In one embodiment, the nanoparticles comprise the colchicine or thiocolchicine dimer and an albumin, preferably colchicine or thiocolchicine dimer coated with the albumin.

In one embodiment, the average diameter of the nanoparticles in the composition is no greater than 200 nm.

In one embodiment, the taxane is in the form of nanoparticles.

In one embodiment, the taxane nanoparticle comprises taxane and an albumin, preferably taxane coated with the albumin.

In one embodiment, the average diameter of the taxane nanoparticles is no greater than 200 nm. In one embodiment, the taxane is paclitaxel.

In one embodiment, the colchicine or thiocolchicine dimer, the anti-VEGF antibody and the taxane are administered concurrently or simultaneously.

In one embodiment, the method comprises intravenously administering at least one of the colchicine or thiocolchicine dimer, the anti-VEGF antibody, and the taxane.

In one embodiment, the anti-VEGF antibody is bevacizumab.

In one embodiment, the proliferative disease is cancer, preferably a vascular tumor, or any of breast cancer, colon cancer, or prostate cancer, or a solid tumor.

In one embodiment, the individual is a human.

The present invention provides compositions of the invention for use in methods for the treatment of proliferative diseases such as cancer. In some embodiments, there is provided a composition for use in a method of treating a proliferative disease in an individual (such as human), wherein the composition comprises a colchicine or thiocolchicine dimer, wherein the method comprises further administration of an effective amount of anti-VEGF antibody and a taxane. The description below focuses on colchicine or thiocolchicine dimer. In some embodiments, the colchicine or thiocochicine dimer is IDN-5404.

In some embodiments, there is provided a composition for use according to any one of the composition for use described above, wherein the colchicine or thiocolchicine dimer is in the form of nanoparticles, such as nanoparticles comprising the colchicine or thiocolchicine dimer and a carrier protein (such as albumin), for example nanoparticles comprising the colchicine or thiocolchicine dimer coated with the carrier protein (such as albumin). In some embodiments, the average diameter of the nanoparticles in the composition is no greater than about 200 nm (such as no greater than about 100 nm).

In some embodiments, there is provided a composition for use according to any one of the composition for use described above, wherein the taxane is in the form of nanoparticles, such as nanoparticle composition comprises a taxane and a carrier protein (such as albumin), for example nanoparticles comprising the taxane coated with the carrier protein (such as albumin). In some embodiments, the average diameter of the nanoparticles in the taxane composition is no greater than about 200 nm. In some embodiments, the taxane is paclitaxel.

In some embodiments, there is provided a composition for use according to any one of the composition for use described above, wherein the colchicine or thiocolchicine dimer and the anti-VEGF antibody are administered concurrently. In some embodiments, the colchicine or thiocolchicine dimer and the anti-VEGF antibody are administered simultaneously. In some embodiments, the colchicine or thiocolchicine dimer is administered no greater than about 24 hours (such as no greater than about any one of 24, 12, 6, 5, 4, 3, 2, or 1 hour) prior to the administration of the anti-VEGF antibody.

In some embodiments, there is provided a composition for use according to any one of the composition for use described above, wherein the colchicine or thiocolchicine dimer and the taxane are administered concurrently. In some embodiments, the colchicine or thiocolchicine dimer and the taxane are administered simultaneously. In some embodiments, the colchicine or thiocolchicine dimer is administered no greater than about 24 hours (such as no greater than about any one of 24, 12, 6, 5, 4, 3, 2, or 1 hour) prior to the administration of the taxane.

In some embodiments, there is provided a composition for use according to any one of the composition for use described above, wherein the colchicine or thiocolchicine dimer is administered intravenously.

In some embodiments, there is provided a composition for use according to any one of the composition for use described above, wherein the anti-VEGF antibody is administered

In some embodiments, there is provided a composition for use according to any one of the composition for use described above, wherein the taxane is administered intravenously.

In some embodiments, there is provided a composition for use according to any one of the composition for use described above, wherein the anti-VEGF antibody is bevacizumab.

In some embodiments, there is provided a composition for use according to any one of the composition for use described above, wherein the proliferative disease is cancer. In some embodiments, the cancer is a vascular tumor. In some embodiments, the cancer is any one of breast cancer, colon cancer, or prostate cancer. In some embodiments, the cancer is a solid tumor.

Also provided are kits and compositions (such as pharmaceutical compositions) useful for methods described herein.

It is understood that aspect and embodiments of the invention described herein include "consisting" and/or "consisting essentially of" aspects and embodiments.

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise.

These and other aspects and advantages of the present invention will become apparent from the subsequent detailed description and the appended claims. It is to be understood that one, some, or all of the properties of the various embodiments described herein may be combined to form other embodiments of the present invention.

### BRIEF DESCRIPTION OF FIGURES

Figure 1 (as reference) shows the effect of sequence of administration of ABI-011 and Abraxane® in the MDA-MB-231 xenograft model.
Figure 2 shows the effect of administration of ABI-011, Abraxane®, and Avastin in the MDA-MB-231 xenograft model.
Figure 3 (as reference) shows the anti-tumor activity of ABI-011 alone in the s.c. human PC3 prostate cancer xenograft model in nude mice.
Figure 4 (as reference) shows the effect of dosage, sequence, and timing of ABI-011 and Abraxane® on the antitumor activity and body weight change in PC3 tumor-bearing mice.
Figure 5 (as reference) shows the effect of dosage, sequence, and timing of ABI-011 and Avastin on the antitumor activity and body weight change in PC3.
Figure 6 (as reference) shows the antitumor activity of ABI-011 plus Avastin in the HT29 xenograft model.
Figure 7 shows the antitumor effect of ABI-011 combinations with Abraxane® and Avastin in the HT29 colon cancer xenograft model.
Figure 8 shows the antitumor effect of ABI-011 combination with Avastin and increasing doses of Abraxane® in the HT29 colon cancer xenograft model.
Figure 9 (as reference) shows antitumor effect of ABI-011 alone and in combination with Avastin in the HT29 colon cancer xenograft model.

### DETAILED DESCRIPTION OF THE INVENTION

The present invention provides a composition for use in a method of treating proliferative disease in an individual, wherein the composition comprises a colchicine or thiocolchicine dimer (such as nanoparticles comprising a colchicine or thiocolchicine dimer and a carrier protein for example albumin), wherein the method comprises further administration of an anti-VEGF antibody and a taxane (such as nanoparticles comprising a taxane or thiocolchicine dimer and a carrier protein for example albumin). In some embodiments, the colchicine or thiocolchicine dimer and the taxane are administered simultaneously. In some embodiments, the colchicine or thiocolchicine dimer is administered no greater than about 24 hours prior to the administration of the taxane.

The present invention is based on the finding that a nanoparticle composition comprising albumin and thiocolchicine dimer 5404 ("Nab-5404" or "ABI-011") in combination with an anti-VEGF antibody (bevacizumab, or Avastin) and/or with a nanoparticle composition comprising albumin and paclitaxel ("nab-paclitaxel" or "Abraxane®") demonstrated significant tumor growth inhibition (TGI) in mouse xenograft models. The optimal therapeutic efficacy was achieved when ABI-011 was administered 24 hours before nab-paclitaxel or concurrent with bevacizumab. Prior to the present invention, it was generally believed that colchicine or thiocolchicine dimer functionally competes with an ant-VEGF antibody. The combination data described here suggest that the combination of the colchicine or thiocolchicine dimer and an anti-VEGF antibody (and/or a taxane) is more effective than monotherapy.

### Methods of combination therapy

The present invention provides compositions of the invention for use in methods for the treatment of proliferative diseases such as cancer. In some embodiments, the cancer is breast cancer, such as metastatic breast cancer. In some embodiments, the cancer is colon cancer. In some embodiments, the cancer is prostate cancer. In some embodiments, the cancer is a vascular tumor, including, for example, medullary carcinoma of the thyroid, angiosarcoma, hemangioendothelioma, hemangioma and Kaposi's sarcoma.

In some embodiments, the method of treating a proliferative disease in an individual comprises administering to the individual: a) an effective amount of a colchicine or thiocolchicine dimer, b) an effective amount of anti-VEGF antibody, and c) an effective amount of a taxane. In some embodiments, the method of treating a proliferative disease in an individual comprises administering to the individual: a) an effective amount of a colchicine or thiocolchicine dimer, b) an effective amount of anti-VEGF antibody, and c) an effective amount of a composition comprising nanoparticles comprising a taxane and a carrier protein (such as albumin). In some embodiments, the proliferative disease (such as cancer) is resistant to the treatment of taxane when administered alone. In some embodiments, the anti-VEGF antibody is bevacizumab (such as Avastin®). In some embodiments, the taxane is paclitaxel and the anti-VEGF antibody is bevacizumab (such as Avastin®). In some embodiments, the colchicine or thiocolchicine dimer, the anti-VEGF antibody, and the taxane are administered intravenously.

In some embodiments, the method of treating a proliferative disease (such as cancer) in an individual comprises administering to the individual: a) an effective amount of a composition comprising nanoparticles comprising colchicine or thiocolchicine dimer, and b) an effective amount of anti-VEGF antibody and c) an effective amount of a taxane. In some embodiments, the method of treating a proliferative disease (such as cancer) in an individual comprises administering to the individual: a) an effective amount of a composition comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin).

In some embodiments, the colchicine or thiocolchicine dimer and the anti-VEGF antibody are administered simultaneously, either in the same composition or in separate compositions. In some embodiments, the colchicine or thiocolchicine dimer and the anti-VEGF antibody are administered sequentially, i.e., the colchicine or thiocolchicine dimer is administered either prior to or after the administration of the anti-VEGF antibody. In some embodiments, the administration of the colchicine or thiocolchicine dimer and the anti-VEGF antibody (or taxane) are concurrent, i.e., the administration period of the colchicine or thiocolchicine dimer and that of the anti-VEGF antibody (or the taxane) overlap with each other.

In some embodiments, the colchicine or thiocolchicine dimer is administered for at least one cycle (for example, at least any of 2, 3, or 4 cycles) prior to the administration of the anti-VEGF antibody or the taxane. In some embodiments, the anti-VEGF antibody (or the taxane) is administered for at least any of one, two, three, or four weeks. In some embodiments, the administrations of the colchicine or thiocolchicine dimer and the anti-VEGF antibody (or the taxane) are initiated at about the same time (for example, within any one of 1, 2, 3, 4, 5, 6, or 7 days). In some embodiments, the administrations of the colchicine or thiocolchicine dimer and the anti-VEGF antibody (or taxane) are terminated at about the same time (for example, within any one of 1, 2, 3, 4, 5, 6, or 7 days). In some embodiments, the administration of the anti-VEGF antibody (or taxane) continues (for example for about any one of 1, 2, 3, 4, 5, 6, 7, 8, 9, 10, 11, or 12 months) after the termination of the administration of the colchicine or thiocolchicine dimer. In some embodiments, the administration of the anti-VEGF antibody (or taxane) is initiated after (for example after about any one of 1, 2, 3, 4, 5, 6, 7, 8, 9, 10, 11, or we months) the initiation of the administration of the colchicine or thiocolchicine dimer. In some embodiments, the administrations of the colchicine or thiocolchicine dimer and the anti-VEGF antibody (or taxane) are initiated and terminated at about the same time. In some embodiments, the administrations of the colchicine or thiocolchicine dimer and the anti-VEGF antibody (or taxane) are initiated at about the same time and the administration of the other agent continues (for example for about any one of 1, 2, 3, 4, 5, 6, 7, 8, 9, 10, 11, or 12 months) after the termination of the administration of the nanoparticle composition. In some embodiments, the administration of the colchicine or thiocolchicine dimer and the anti-VEGF antibody (or taxane) stop at about the same time and the administration of the anti-VEGF antibody (or taxane) is initiated after (for example after about any one of 1, 2, 3, 4, 5, 6, 7, 8, 9, 10, 11, or we months) the initiation of the administration of the nanoparticle composition.

In some embodiments, the administration of the colchicine or thiocolchicine dimer and the anti-VEGF antibody (or taxane) are non-concurrent. For example, in some embodiments, the administration of the colchicine or thiocolchicine dimer is terminated before the anti-VEGF antibody (or taxane) is administered. In some embodiments, the administration of the anti-VEGF antibody (or taxane) is terminated before the colchicine or thiocolchicine dimer is administered. The time period between these two non-concurrent administrations can range from about two to eight weeks, such as about four weeks.

Also disclosed is a method of treating a proliferative disease (such as cancer) in an individual comprising administering to the individual: a) an effective amount of a colchicine or thiocolchicine dimer, and b) an effective amount of anti-VEGF antibody, wherein the colchicine or thiocolchicine dimer and the anti-VEGF antibody are administered simultaneously. Also disclosed is a method of treating a proliferative disease (such as cancer) in an individual comprising administering to the individual: a) an effective amount of a colchicine or thiocolchicine dimer, and b) an effective amount of anti-VEGF antibody, wherein the colchicine or thiocolchicine dimer and the anti-VEGF antibody are administered concurrently. In some examples, there is a method of treating a proliferative disease (such as cancer) in an individual comprising administering to the individual: a) an effective amount of a colchicine or thiocolchicine dimer, and b) an effective amount of anti-VEGF antibody, wherein the colchicine or thiocolchicine dimer is administered no greater than about 24 hours (such as 24 hours) prior to the administration of the anti-VEGF antibody.

Also disclosed is a method of treating a proliferative disease (such as cancer) in an individual comprising administering to the individual: a) an effective amount of a composition comprising a colchicine or thiocolchicine dimer and a carrier protein; and b) an effective of an anti-VEGF antibody. In some examples, there is a method of treating a proliferative disease (such as cancer) in an individual comprising administering to the individual: a) an effective amount of a composition comprising a colchicine or thiocolchicine dimer and a carrier protein; and b) an effective amount of anti-VEGF antibody, wherein the colchicine or thiocolchicine dimer and the anti-VEGF antibody are administered simultaneously. In some examples, there is a method of treating a proliferative disease (such as cancer) in an individual comprising administering to the individual: a) an effective amount of a composition comprising a colchicine or thiocolchicine dimer and a carrier protein; and b) an effective amount of anti-VEGF antibody, wherein the colchicine or thiocolchicine dimer and the anti-VEGF antibody are administered concurrently. In some examples, there is a method of treating a proliferative disease (such as cancer) in an individual comprising administering to the individual: a) an effective amount of a composition comprising a colchicine or thiocolchicine dimer and a carrier protein; and b) an effective amount of anti-VEGF antibody, wherein the colchicine or thiocolchicine dimer is administered no greater than about 24 hours (such as 24 hours) prior to the administration of the anti-VEGF antibody.

Also disclosed is a method of treating a proliferative disease (such as cancer) in an individual comprising administering to the individual: a) an effective amount of a composition comprising nanoparticles comprising a colchicine or thiocolchicine dimer and a carrier protein (such as albumin); and b) an effective of an anti-VEGF antibody. Also disclosed is a method of treating a proliferative disease (such as cancer) in an individual comprising administering to the individual: a) an effective amount of a composition comprising nanoparticles comprising a colchicine or thiocolchicine dimer and a carrier protein (such as albumin); and b) an effective amount of anti-VEGF antibody, wherein the colchicine or thiocolchicine dimer and the anti-VEGF antibody are administered simultaneously. In some examples, there is a method of treating a proliferative disease (such as cancer) in an individual comprising administering to the individual: a) an effective amount of a composition comprising nanoparticles comprising a colchicine or thiocolchicine dimer and a carrier protein (such as albumin); and b) an effective amount of anti-VEGF antibody, wherein the colchicine or thiocolchicine dimer and the anti-VEGF antibody are administered concurrently. In some examples, there is a method of treating a proliferative disease (such as cancer) in an individual comprising administering to the individual: a) an effective amount of a composition comprising nanoparticles comprising a colchicine or thiocolchicine dimer and a carrier protein (such as albumin); and b) an effective amount of anti-VEGF antibody, wherein the colchicine or thiocolchicine dimer is administered no greater than about 24 hours (such as 24 hours) prior to the administration of the anti-VEGF antibody.

Also disclosed is a method of treating a proliferative disease (such as cancer) in an individual comprising administering to the individual: a) an effective amount of a composition comprising nanoparticles comprising a colchicine or thiocolchicine dimer coated with a carrier protein (such as albumin); and b) an effective of an anti-VEGF antibody. Also disclosed is a method of treating a proliferative disease (such as cancer) in an individual comprising administering to the individual: a) an effective amount of a composition comprising nanoparticles comprising a colchicine or thiocolchicine dimer coated with a carrier protein (such as albumin); and b) an effective amount of anti-VEGF antibody, wherein the colchicine or thiocolchicine dimer and the anti-VEGF antibody are administered simultaneously. In some examples, there is a method of treating a proliferative disease (such as cancer) in an individual comprising administering to the individual: a) an effective amount of a composition comprising nanoparticles comprising a colchicine or thiocolchicine dimer coated with a carrier protein (such as albumin); and b) an effective amount of anti-VEGF antibody, wherein the colchicine or thiocolchicine dimer and the anti-VEGF antibody are administered concurrently. In some examples, there is a method of treating a proliferative disease (such as cancer) in an individual comprising administering to the individual: a) an effective amount of a composition comprising nanoparticles comprising a colchicine or thiocolchicine dimer coated with a carrier protein (such as albumin); and b) an effective amount of anti-VEGF antibody, wherein the colchicine or thiocolchicine dimer is administered no greater than about 24 hours (such as 24 hours) prior to the administration of the anti-VEGF antibody.

In some embodiments, the colchicine or thiocolchicine dimer and the taxane are administered simultaneously, either in the same composition or in separate compositions. In some embodiments, the colchicine or thiocolchicine dimer and the taxane are administered sequentially, i.e., the colchicine or thiocolchicine dimer is administered either prior to or after the administration of the taxane. In some embodiments, the colchicine or thiocolchicine dimer is administered no greater than about 24 hours (for example 24 hours) prior to the administration of the taxane. In some embodiments, the administration of the colchicine or thiocolchicine dimer and the taxane are concurrent, i.e., the administration period of the colchicine or thiocolchicine dimer and that of the taxane overlap with each other.

Thus, in some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a taxane, wherein at least two of the colchicine or thiocolchicine dimer, the anti-VEGF antibody, and the taxane are administered simultaneously. In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a composition comprising nanoparticles comprising a taxane and a carrier protein (such as albumin), wherein at least two of the colchicine or thiocolchicine dimer, the anti-VEGF antibody, and the taxane are administered simultaneously. In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a composition comprising nanoparticles comprising a taxane and a carrier protein (such as albumin), wherein at least two of the colchicine or thiocolchicine dimer, the anti-VEGF antibody, and the taxane are administered simultaneously by intravenous administration.

In some embodiments, the method of treating a proliferative disease (such as cancer) in an individual comprises intravenously administering to the individual: a) an effective amount of a colchicine or thiocolchicine dimer, b) an effective amount of anti-VEGF antibody, and c) an effective amount of a taxane. In some embodiments, the method of treating a proliferative disease (such as cancer) in an individual comprises intravenously administering to the individual: a) an effective amount of a colchicine or thiocolchicine dimer, b) an effective amount of anti-VEGF antibody, and c) an effective amount of a taxane, wherein at least two of the colchicine or thiocolchicine dimer, the anti-VEGF antibody, and the taxane are administered simultaneously. In some embodiments, the colchicine or thiocolchicine dimer, the anti-VEGF antibody, and the taxane are administered simultaneously.

In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a taxane, wherein the colchicine or thiocolchicine dimer and the taxane are administered simultaneously. In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a composition comprising nanoparticles comprising a taxane and a carrier protein (such as albumin), wherein the colchicine or thiocolchicine dimer and the taxane are administered simultaneously.

In some embodiments, the method of treating a proliferative disease (such as cancer) in an individual comprises administering to the individual: a) an effective amount of a composition comprising a colchicine or thiocolchicine dimer, b) an effective amount of anti-VEGF antibody, and c) an effective amount of a taxane, wherein the colchicine or thiocolchicine dimer and the taxane are administered simultaneously.

In some embodiments, the method of treating a proliferative disease (such as cancer) in an individual comprises administering to the individual: a) an effective amount of a composition comprising a colchicine or thiocolchicine dimer and a carrier protein (such as albumin), b) an effective amount of anti-VEGF antibody, and c) an effective amount of a taxane, wherein the colchicine or thiocolchicine dimer and the taxane are administered simultaneously. In some embodiments, the method of treating a proliferative disease (such as cancer) in an individual comprises administering to the individual: a) an effective amount of a composition comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer and the taxane are administered simultaneously.

In some embodiments, the method of treating a proliferative disease (such as cancer) in an individual comprises administering to the individual: a) an effective amount of a composition comprising nanoparticles comprising a colchicine or thiocolchicine dimer and a carrier protein (such as albumin), b) an effective amount of anti-VEGF antibody, and c) an effective amount of a taxane, wherein the colchicine or thiocolchicine dimer and the taxane are administered simultaneously. In some embodiments, the method of treating a proliferative disease (such as cancer) in an individual comprises administering to the individual: a) an effective amount of composition comprising nanoparticles comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer and the taxane are administered simultaneously. In some embodiments, the method of treating a proliferative disease (such as cancer) in an individual comprises administering to the individual: a) an effective amount of a nanoparticle composition comprising colchicine or thiocolchicine dimer coated with a carrier protein (such as albumin), b) an effective amount of anti-VEGF antibody, and c) an effective amount of a composition comprising nanoparticles comprising a taxane coated with a carrier protein (such as albumin), wherein the colchicine or thiocolchicine dimer and the taxane are administered simultaneously.

In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a taxane, wherein the colchicine or thiocolchicine dimer and the taxane are administered concurrently. In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a composition comprising nanoparticles comprising a taxane and a carrier protein (such as albumin), wherein the colchicine or thiocolchicine dimer and the taxane are administered concurrently.

In some embodiments, the method of treating a proliferative disease (such as cancer) in an individual comprises administering to the individual: a) an effective amount of a composition comprising a colchicine or thiocolchicine dimer, b) an effective amount of anti-VEGF antibody, and c) an effective amount of a taxane, wherein the colchicine or thiocolchicine dimer and the taxane are administered concurrently.

In some embodiments, the method of treating a proliferative disease (such as cancer) in an individual comprises administering to the individual: a) an effective amount of a composition comprising a colchicine or thiocolchicine dimer and a carrier protein (such as albumin), b) an effective amount of anti-VEGF antibody, and c) an effective amount of a taxane, wherein the colchicine or thiocolchicine dimer and the taxane are administered concurrently. In some embodiments, the method of treating a proliferative disease (such as cancer) in an individual comprises administering to the individual: a) an effective amount of a composition comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer and the taxane are administered concurrently.

In some embodiments, the method of treating a proliferative disease (such as cancer) in an individual comprises administering to the individual: a) an effective amount of a composition comprising nanoparticles comprising a colchicine or thiocolchicine dimer and a carrier protein (such as albumin), b) an effective amount of anti-VEGF antibody, and c) an effective amount of a taxane, wherein the colchicine or thiocolchicine dimer and the taxane are administered concurrently. In some embodiments, the method of treating a proliferative disease (such as cancer) in an individual comprises administering to the individual: a) an effective amount of a composition comprising nanoparticles comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer and the taxane are administered concurrently. In some embodiments, the method of treating a proliferative disease (such as cancer) in an individual comprises administering to the individual: a) an effective amount of a composition comprising nanoparticles comprising colchicine or thiocolchicine dimer coated with a carrier protein (such as albumin), b) an effective amount of anti-VEGF antibody, and c) an effective amount of a composition comprising nanoparticles comprising a taxane coated with a carrier protein (such as albumin), wherein the colchicine or thiocolchicine dimer and the taxane are administered concurrently.

In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a taxane, wherein the colchicine or thiocolchicine dimer is administered prior to (such as no greater than about 24 hours prior to) the administration of the taxane. In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a composition comprising nanoparticles comprising a taxane and a carrier protein (such as albumin), wherein the colchicine or thiocolchicine dimer is administered prior to (such as no greater than about 24 hours prior to) the administration of the taxane.

In some embodiments, the method of treating a proliferative disease (such as cancer) in an individual comprises administering to the individual: a) an effective amount of a composition comprising a colchicine or thiocolchicine dimer, b) an effective amount of anti-VEGF antibody, and c) an effective amount of a taxane, wherein the colchicine or thiocolchicine dimer is administered prior to (such as no greater than about 24 hours prior to) the administration of the taxane. In some embodiments, the method of treating a proliferative disease (such as cancer) in an individual comprises administering to the individual: a) an effective amount of a composition comprising a colchicine or thiocolchicine dimer and a carrier protein, b) an effective amount of anti-VEGF antibody, and c) an effective amount of a taxane, wherein the colchicine or thiocolchicine dimer is administered prior to (such as no greater than about 24 hours prior to) the administration of the taxane. In some embodiments, the method of treating a proliferative disease (such as cancer) in an individual comprises administering to the individual: a) an effective amount of a composition comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer is administered prior to (such as no greater than about 24 hours prior to) the administration of the taxane.

In some embodiments, the method of treating a proliferative disease (such as cancer) in an individual comprises administering to the individual: a) an effective amount of a composition comprising nanoparticles comprising a colchicine or thiocolchicine dimer and a carrier protein, b) an effective amount of anti-VEGF antibody, and c) an effective amount of a taxane, wherein the colchicine or thiocolchicine dimer is administered prior to (such as no greater than about 24 hours prior to) the administration of the taxane. In some embodiments, the method of treating a proliferative disease (such as cancer) in an individual comprises administering to the individual: a) an effective amount of a composition comprising nanoparticles comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer is administered prior to (such as no greater than about 24 hours prior to) the administration of the taxane. In some embodiments, the method of treating a proliferative disease (such as cancer) in an individual comprises administering to the individual: a) an effective amount of a composition comprising nanoparticles comprising colchicine or thiocolchicine dimer coated with a carrier protein (such as albumin), b) an effective amount of anti-VEGF antibody, and c) an effective amount of a composition comprising nanoparticles comprising a taxane coated with a carrier protein (such as albumin), wherein the colchicine or thiocolchicine dimer is administered prior to (such as no greater than about 24 hours prior to) the administration of the taxane.

In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a taxane, wherein the colchicine or thiocolchicine dimer, the anti-VEGF antibody, and the taxane are administered simultaneously. In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a composition comprising nanoparticles comprising a taxane and a carrier protein (such as albumin), wherein the colchicine or thiocolchicine dimer, the anti-VEGF antibody, and the taxane are administered simultaneously.

In some embodiments, the method of treating a proliferative disease (such as cancer) in an individual comprises administering to the individual: a) an effective amount of a composition comprising a colchicine or thiocolchicine dimer, b) an effective amount of anti-VEGF antibody, and c) an effective amount of a taxane, wherein the colchicine or thiocolchicine dimer, the anti-VEGF antibody, and the taxane are administered simultaneously. In some embodiments, the method of treating a proliferative disease (such as cancer) in an individual comprises administering to the individual: a) an effective amount of a composition comprising a colchicine or thiocolchicine dimer and a carrier protein, b) an effective amount of anti-VEGF antibody, and c) an effective amount of a taxane, wherein the colchicine or thiocolchicine dimer, the anti-VEGF antibody, and the taxane are administered simultaneously. In some embodiments, the method of treating a proliferative disease (such as cancer) in an individual comprises administering to the individual: a) an effective amount of a composition comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer, the anti-VEGF antibody, and the taxane are administered simultaneously.

In some embodiments, the method of treating a proliferative disease (such as cancer) in an individual comprises administering to the individual: a) an effective amount of a composition comprising nanoparticles comprising a colchicine or thiocolchicine dimer and a carrier protein, b) an effective amount of anti-VEGF antibody, and c) an effective amount of a taxane, wherein the colchicine or thiocolchicine dimer, the anti-VEGF antibody, and the taxane are administered simultaneously. In some embodiments, the method of treating a proliferative disease (such as cancer) in an individual comprises administering to the individual: a) an effective amount of a composition comprising nanoparticles comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer, the anti-VEGF antibody, and the taxane are administered simultaneously. In some embodiments, the method of treating a proliferative disease (such as cancer) in an individual comprises administering to the individual: a) an effective amount of a composition comprising nanoparticles comprising colchicine or thiocolchicine dimer coated with a carrier protein (such as albumin), b) an effective amount of anti-VEGF antibody, and c) an effective amount of a composition comprising nanoparticles comprising a taxane coated with a carrier protein (such as albumin), wherein the colchicine or thiocolchicine dimer, the anti-VEGF antibody, and the taxane are administered simultaneously.

In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a taxane, wherein the colchicine or thiocolchicine dimer, the anti-VEGF antibody, and the taxane are administered concurrently. In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a composition comprising nanoparticles comprising a taxane and a carrier protein (such as albumin), wherein the colchicine or thiocolchicine dimer, the anti-VEGF antibody, and the taxane are administered concurrently.

In some embodiments, the method of treating a proliferative disease (such as cancer) in an individual comprises administering to the individual: a) an effective amount of a composition comprising a colchicine or thiocolchicine dimer, b) an effective amount of anti-VEGF antibody, and c) an effective amount of a taxane, wherein the colchicine or thiocolchicine dimer, the anti-VEGF antibody, and the taxane are administered concurrently. In some embodiments, the method of treating a proliferative disease (such as cancer) in an individual comprises administering to the individual: a) an effective amount of a composition comprising a colchicine or thiocolchicine dimer and a carrier protein, b) an effective amount of anti-VEGF antibody, and c) an effective amount of a taxane, wherein the colchicine or thiocolchicine dimer, the anti-VEGF antibody, and the taxane are administered concurrently. In some embodiments, the method of treating a proliferative disease (such as cancer) in an individual comprises administering to the individual: a) an effective amount of a composition comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer, the anti-VEGF antibody, and the taxane are administered concurrently.

In some embodiments, the method of treating a proliferative disease (such as cancer) in an individual comprises administering to the individual: a) an effective amount of a composition comprising nanoparticles comprising a colchicine or thiocolchicine dimer and a carrier protein, b) an effective amount of anti-VEGF antibody, and c) an effective amount of a taxane, wherein the colchicine or thiocolchicine dimer, the anti-VEGF antibody, and the taxane are administered concurrently. In some embodiments, the method of treating a proliferative disease (such as cancer) in an individual comprises administering to the individual: a) an effective amount of a composition comprising nanoparticles comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer, the anti-VEGF antibody, and the taxane are administered concurrently. In some embodiments, the method of treating a proliferative disease (such as cancer) in an individual comprises administering to the individual: a) an effective amount of a composition comprising nanoparticles comprising colchicine or thiocolchicine dimer coated with a carrier protein (such as albumin), b) an effective amount of anti-VEGF antibody, and c) an effective amount of a composition comprising nanoparticles comprising a taxane coated with a carrier protein (such as albumin), wherein the colchicine or thiocolchicine dimer, the anti-VEGF antibody, and the taxane are administered concurrently.

In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a taxane, wherein the colchicine or thiocolchicine dimer is administered prior to (such as no greater than about 24 hours prior to) the administration of the taxane, and wherein the colchicine and thiocolchicine dimer is administered concurrently with the anti-VEGF antibody. In some embodiments, the method of treating a proliferative disease (such as cancer) in an individual comprises administering to the individual: a) an effective amount of a colchicine or thiocolchicine dimer, b) an effective amount of anti-VEGF antibody, and c) an effective amount of a composition comprising nanoparticles comprising a taxane and a carrier protein (such as albumin), wherein the colchicine or thiocolchicine dimer is administered prior to (such as no greater than about 24 hours prior to) the administration of the taxane, and wherein the colchicine and thiocolchicine dimer is administered concurrently with the anti-VEGF antibody.

In some embodiments, the method of treating a proliferative disease (such as cancer) in an individual comprises administering to the individual: a) an effective amount of a composition comprising a colchicine or thiocolchicine dimer, b) an effective amount of anti-VEGF antibody, and c) an effective amount of a taxane, wherein the colchicine or thiocolchicine dimer is administered prior to (such as no greater than about 24 hours prior to) the administration of the taxane, and wherein the colchicine and thiocolchicine dimer is administered concurrently with the anti-VEGF antibody. In some embodiments, the method of treating a proliferative disease (such as cancer) in an individual comprises administering to the individual: a) an effective amount of a composition comprising a colchicine or thiocolchicine dimer and a carrier protein, b) an effective amount of anti-VEGF antibody, and c) an effective amount of a taxane, wherein the colchicine or thiocolchicine dimer is administered prior to (such as no greater than about 24 hours prior to) the administration of the taxane, and wherein the colchicine and thiocolchicine dimer is administered concurrently with the anti-VEGF antibody. In some embodiments, the method of treating a proliferative disease (such as cancer) in an individual comprises administering to the individual: a) an effective amount of a composition comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer is administered prior to (such as no greater than about 24 hours prior to) the administration of the taxane, and wherein the colchicine and thiocolchicine dimer is administered concurrently with the anti-VEGF antibody.

In some embodiments, the method of treating a proliferative disease (such as cancer) in an individual comprises administering to the individual: a) an effective amount of a composition comprising nanoparticles comprising a colchicine or thiocolchicine dimer and a carrier protein, b) an effective amount of anti-VEGF antibody, and c) an effective amount of a taxane, wherein the colchicine or thiocolchicine dimer is administered prior to (such as no greater than about 24 hours prior to) the administration of the taxane, and wherein the colchicine and thiocolchicine dimer is administered concurrently with the anti-VEGF antibody. In some embodiments, the method of treating a proliferative disease (such as cancer) in an individual comprises administering to the individual: a) an effective amount of a composition comprising nanoparticles comprising colchicine or thiocolchicine dimer and a carrier protein (such as albumin), b) an effective amount of anti-VEGF antibody, and c) an effective amount of a composition comprising nanoparticles comprising a taxane and a carrier protein (such as albumin), wherein the colchicine or thiocolchicine dimer is administered prior to (such as no greater than about 24 hours prior to) the administration of the taxane, and wherein the colchicine and thiocolchicine dimer is administered concurrently with the anti-VEGF antibody. In some embodiments, the method of treating a proliferative disease (such as cancer) in an individual comprises administering to the individual: a) an effective amount of a composition comprising nanoparticles comprising colchicine or thiocolchicine dimer coated with a carrier protein (such as albumin), b) an effective amount of anti-VEGF antibody, and c) an effective amount of a composition comprising nanoparticles comprising a taxane coated with a carrier protein (such as albumin), wherein the colchicine or thiocolchicine dimer is administered prior to (such as no greater than about 24 hours prior to) the administration of the taxane, and wherein the colchicine and thiocolchicine dimer is administered concurrently with the anti-VEGF antibody.

The methods in some embodiments comprise administration of a composition comprising nanoparticles comprising a colchicine or thiocolchicine dimer (or a taxane) and a carrier protein. In some embodiments, the nanoparticle composition comprises nanoparticles comprising a colchicine or thiocolchicine dimer (or a taxane) and an albumin.

In some embodiments, the nanoparticles in the compositions described herein have an average diameter of no greater than about 200 nm, including for example no greater than about any one of 190, 180, 170, 160, 150, 140, 130, 120, 110, 100, 90, 80, 70, or 60 nm. In some embodiments, the nanoparticles in the compositions described herein have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of all the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of all the nanoparticles in the composition fall within the range of about 20 to about 200 nm, including for example any one of about 30 to about 180 nm, and any one of about 40 to about 150, about 50 to about 120, and about 60 to about 100 nm.

In some embodiments, the carrier protein has sulfhydral groups that can form disulfide bonds. In some embodiments, at least about 5% (including for example at least about any one of 10%, 15%, 20%, 25%, 30%, 40%, 50%, 60%, 70%, 80%, or 90%) of the carrier protein in the nanoparticle portion of the composition are crosslinked (for example crosslinked through one or more disulfide bonds).

In some embodiments, the nanoparticles comprise the colchicine or thiocolchicine dimer (or a taxane) coated with a carrier protein, such as albumin (e.g., human serum albumin). In some embodiments, the composition comprises a colchicine or thiocolchicine dimer (or a taxane) in both nanoparticle and non-nanoparticle forms, wherein at least about any one of 50%, 60%, 70%, 80%, 90%, 95%, or 99% of the drug in the composition are in nanoparticle form. In some embodiments, the taxane in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of the drug that is substantially free of polymeric materials (such as polymeric matrix).

In some embodiments, the nanoparticle composition is substantially free (such as free) of surfactants (such as Cremophor®, Tween 80, or other organic solvents used for the administration of colchicine or thiocolchicine dimer or taxanes). In some embodiments, the nanoparticle composition contains less than about any one of 20%, 15%, 10%, 7.5%, 5%, 2.5%, or 1% organic solvent. In some embodiments, the weight ratio of carrier protein (such as albumin) and colchicine or thiocolchicine dimer (or taxane) in the nanoparticle composition is about 18:1 or less, such as about 15:1 or less, for example about 10:1 or less. In some embodiments, the weight ratio of carrier protein (such as albumin) and colchicine or thiocolchicine dimer (or taxane) in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of carrier protein and colchicine or thiocolchicine dimer (or taxane) in the nanoparticle portion of the composition is about any one of 1:2, 1:3, 1:4, 1:5, 1:10, 1:15, or less.

In some embodiments, the particle composition comprises one or more of the above characteristics.

As disclosed herein is a method of treating a proliferative disease (such as cancer) in an individual comprising administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), and b) an effective amount of an anti-VEGF antibody (such as bevacizumab (for example Avastin®). In some embodiments, the method of treating a proliferative disease (such as cancer) in an individual comprises administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), b) an effective amount of an anti-VEGF antibody (such as bevacizumab (for example Avastin®), and c) an effective amount of a composition comprising nanoparticles comprising a taxane (such as paclitaxel) coated with albumin, wherein the nanoparticles have an average diameter of no greater than about 200 nm.

As disclosed herein is a method of treating a proliferative disease (such as cancer) in an individual comprising administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), and b) an effective amount of an anti-VEGF antibody (such as bevacizumab (for example Avastin®). Also disclosed herein is a method of treating a proliferative disease (such as cancer) in an individual comprising administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), and b) an effective amount of an anti-VEGF antibody (such as bevacizumab (for example Avastin®), wherein the composition comprising nanoparticles comprising the colchicine or thiocolchicine dimer and the anti-VEGF antibody are administered concurrently. Also disclosed herein is provided a method of treating a proliferative disease (such as cancer) in an individual comprising administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), and b) an effective amount of an anti-VEGF antibody (such as bevacizumab (for example Avastin®), wherein the composition comprising nanoparticles comprising the colchicine or thiocolchicine dimer and the anti-VEGF antibody are administered simultaneously. Also disclosed herein is a method of treating a proliferative disease (such as cancer) in an individual comprising administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), and b) an effective amount of an anti-VEGF antibody (such as bevacizumab (for example Avastin®), wherein the composition comprising nanoparticles comprising the colchicine or thiocolchicine dimer is administered no greater than about 24 hours (such as 24 hours) prior to the administration of the anti-VEGF antibody.

In some embodiments, the method of treating a proliferative disease (such as cancer) in an individual comprises administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), b) an effective amount of an anti-VEGF antibody (such as bevacizumab (for example Avastin®), and c) an effective amount of a composition comprising nanoparticles comprising a taxane (such as paclitaxel) coated with albumin, wherein the nanoparticles have an average diameter of no greater than about 200 nm (such as no greater than about 100 nm). In some embodiments, the method of treating a proliferative disease (such as cancer) in an individual comprises administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), b) an effective amount of an anti-VEGF antibody (such as bevacizumab (for example Avastin®), and c) an effective amount of a composition comprising nanoparticles comprising a taxane (such as paclitaxel) coated with albumin, wherein the nanoparticles have an average diameter of no greater than about 200 nm, wherein the composition comprising nanoparticles comprising the colchicine or thiocolchicine dimer, the anti-VEGF antibody, and the composition comprising the taxane are administered simultaneously. In some embodiments, the method of treating a proliferative disease (such as cancer) in an individual comprises administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), b) an effective amount of an anti-VEGF antibody (such as bevacizumab (for example Avastin®), and c) an effective amount of a composition comprising nanoparticles comprising a taxane (such as paclitaxel) coated with albumin, wherein the nanoparticles have an average diameter of no greater than about 200 nm, wherein the composition comprising nanoparticles comprising the colchicine or thiocolchicine dimer, the anti-VEGF antibody, and the composition comprising the taxane are administered concurrently.

In some embodiments, the method of treating a proliferative disease (such as cancer) in an individual comprises administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), b) an effective amount of an anti-VEGF antibody (such as bevacizumab (for example Avastin®), and c) an effective amount of a composition comprising nanoparticles comprising a taxane (such as paclitaxel) coated with albumin, wherein the nanoparticles have an average diameter of no greater than about 200 nm, wherein the composition comprising nanoparticles comprising the colchicine or thiocolchicine dimer is administered prior to (such as no greater than about 24 hours prior to) the composition comprising the taxane, and wherein the composition comprising the colchicine or thiocolchicine dimer are administered concurrently.

In some embodiments, the method of treating a proliferative disease (such as cancer) in an individual comprises intravenously administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), b) an effective amount of an anti-VEGF antibody (such as bevacizumab (for example Avastin®), and c) an effective amount of a composition comprising nanoparticles comprising a taxane (such as paclitaxel) coated with albumin, wherein the nanoparticles have an average diameter of no greater than about 200 nm. In some embodiments, the method of treating a proliferative disease (such as cancer) in an individual comprises intravenously and simultaneously administering to the individual: a) an effective amount of a composition comprising nanoparticles comprising a colchicine or thiocolchicine dimer coated with albumin, wherein the nanoparticles have an average diameter of no greater than about 200nm (such as no greater than about 100 nm), b) an effective amount of an anti-VEGF antibody (such as bevacizumab (for example Avastin®), and c) an effective amount of a composition comprising nanoparticles comprising a taxane (such as paclitaxel) coated with albumin, wherein the nanoparticles have an average diameter of no greater than about 200 nm.

As disclosed herein is a method of treating a proliferative disease (such as cancer) in an individual comprising administering to the individual: a) an effective amount of Nab-5404, and b) an effective amount of bevacizumab (such as Avastin®). In some examples, the Nab-5404 and the bevacizumab are administered concurrently. In some examples, the Nab-5404 and the bevacizumab are administered simultaneously. In some examples, the Nab-5404 is administered no greater than about 24 hours prior to the administration of the bevacizumab. In some examples, the bevacizumab is administered no greater than about 24 hours prior to the administration of the Nab-5404.

In some embodiments, the method of treating a proliferative disease (such as cancer) in an individual comprises administering to the individual: a) an effective amount of Nab-5404, b) an effective amount of bevacizumab, and c) an effective amount of Abraxane®. In some embodiments, the Nab-5404 and the Abraxane® are administered simultaneously. In some embodiments, the Nab-5404 and the Abraxane® are administered concurrently. In some embodiments, the Nab-5404 is administered no greater than about 24 hours prior to the administration of the Abraxane®. In some embodiments, the Abraxane® is administered no greater than about 24 hours prior to the administration of the Nab-5404.

In some embodiments, the method of treating a proliferative disease (such as cancer) in an individual comprises intravenously administering to the individual: a) an effective amount of Nab-5404, b) an effective amount of bevacizumab, and c) an effective amount of Abraxane®. In some embodiments, the Nab-5404 and the Abraxane® are administered simultaneously. In some embodiments, the Nab-5404 and the bevacizumab are administered simultaneously. In some embodiments, the Nab-5404, the Abraxane®, and the bevacizumab are administered simultaneously.

In some embodiments, the effective amounts of the colchicine or thiocolchicine dimer and the anti-VEGF antibody synergistically inhibit cell proliferation (such as tumor cell growth). In some embodiments, the effective amounts of the colchicine or thiocolchicine dimer, the anti-VEGF antibody, and the taxane synergistically inhibit cell proliferation (such as tumor cell growth). In some embodiments, at least about 10% (including for example at least about any of 20%, 30%, 40%, 60%, 70%, 80%, 90%, or 100%) cell proliferation is inhibited. In some embodiments, the taxane is paclitaxel.

In some embodiments, the method of treating a proliferative disease (such as cancer, for example breast cancer) in an individual, comprises administering to the individual: a) an effective amount of a colchicine or thiocolchicine dimer; b) an effective amount of an anti-VEGF antibody; and c) an effective amount of a taxane, wherein the colchicine or thiocolchicine dimer and the anti-VEGF antibody are in an amount effective to suppress taxane-mediated upregulation of the pro survival and/or inflammatory signal in vivo.

In some embodiments, the methods further comprise administration of one or more additional agent. In some embodiments, the additional agent is a chemotherapeutic agent, such as chemotherapeutic agents described in U.S. Patent Application No. 2006/0263434. In some embodiments, the additional agent is any one of rapamycin, dexamethasone, bortezomib, imatinib, sorafenib, gemcitabine, lenalidomide, and sunitinib.

The methods described herein are generally useful for treatment of diseases, particularly proliferative diseases. As used herein, "treatment" is an approach for obtaining beneficial or desired clinical results. For purposes of this invention, beneficial or desired clinical results include, any one or more of: alleviation of one or more symptoms, diminishment of extent of disease, preventing or delaying spread (e.g., metastasis, for example metastasis to the lung or to the lymph node) of disease, preventing or delaying recurrence of disease, delay or slowing of disease progression, amelioration of the disease state, and remission (whether partial or total). Also encompassed by "treatment" is a reduction of pathological consequence of a proliferative disease. The uses of the invention contemplate any one or more of these aspects of treatment.

As used herein, a "proliferative disease" is defined as a tumor disease (including benign or cancerous) and/or any metastases, wherever the tumor or the metastasis are located, more specifically a tumor selected from the group comprising one or more of (and in some embodiments selected from the group consisting of) breast cancer, genitourinary cancer, lung cancer, gastrointestinal cancer, epidermoid cancer, melanoma, ovarian cancer, pancreatic cancer, neuroblastoma, colorectal cancer, head and neck cancer. In a broader sense of the invention, a proliferative disease may furthermore be selected from hyperproliferative conditions such as hyperplasias, fibrosis (especially pulmonary, but also other types of fibrosis, such as renal fibrosis), angiogenesis, psoriasis, atherosclerosis and smooth muscle proliferation in the blood vessels, such as stenosis or restenosis following angioplasty. In some embodiments, the proliferative disease is cancer. In some embodiments, the proliferative disease is a non-cancerous disease. In some embodiments, the proliferative disease is a benign or malignant tumor. Where hereinbefore and subsequently a tumor, a tumor disease, a carcinoma or a cancer are mentioned, also metastasis in the original organ or tissue and/or in any other location are implied alternatively or in addition, whatever the location of the tumor and/or metastasis is.

The term "effective amount" used herein refers to an amount of a compound or composition sufficient to treat a specified disorder, condition or disease such as ameliorate, palliate, lessen, and/or delay one or more of its symptoms. In reference to cancers or other unwanted cell proliferation, an effective amount comprises an amount sufficient to cause a tumor to shrink and/or to decrease the growth rate of the tumor (such as to suppress tumor growth) or to prevent or delay other unwanted cell proliferation. In some embodiments, an effective amount is an amount sufficient to delay development. In some embodiments, an effective amount is an amount sufficient to prevent or delay recurrence. An effective amount can be administered in one or more administrations. In the case of cancer, the effective amount of the drug or composition may: (i) reduce the number of cancer cells; (ii) reduce tumor size; (iii) inhibit, retard, slow to some extent and preferably stop cancer cell infiltration into peripheral organs; (iv) inhibit (i.e., slow to some extent and preferably stop) tumor metastasis; (v) inhibit tumor growth; (vi) prevent or delay occurrence and/or recurrence of tumor; and/or (vii) relieve to some extent one or more of the symptoms associated with the cancer. In some embodiments, the amount of the drug(s) is effective to result in a tumor growth inhibition (TGI) of at least about any of 75%, 80%, 85%, 90%, 95%, 99%, or a TGI of 100%.

In some embodiments, the method is for treating a primary tumor. In some embodiments, the method is for treating metastatic cancer (that is, cancer that has metastasized from the primary tumor). In some embodiments, the method is for treating a proliferative disease such as cancer (and in broader aspect method of treating a proliferative disease) at advanced stage(s). In some embodiments, the method is for treating breast cancer (which may be HER2 positive or HER2 negative), including, for example, advanced breast cancer, stage IV breast cancer, locally advanced breast cancer, and metastatic breast cancer. In some embodiments, the method is for treating lung cancer, including, for example, non-small cell lung cancer (NSCLC, such as advanced NSCLC), small cell lung cancer (SCLC, such as advanced SCLC), and advanced solid tumor malignancy in the lung. In some embodiments, the method is for treating any of ovarian cancer, head and neck cancer, gastric malignancies, melanoma (including metastatic melanoma and malignant melanoma), colorectal cancer, pancreatic cancer, and solid tumors (such as advanced solid tumors). In some embodiments, the method is for treating a disease that is refractory to treatment of a taxane when administered alone. In some embodiments, the method is for reducing cell proliferation and/or cell migration. In some embodiments, the method is for treating any of the following diseases: restenosis, stenosis, fibrosis, angiogenesis, psoriasis, atherosclerosis, and proliferation of smooth muscle cells. The present invention also provides compositions of the invention for use in methods of delaying development of any of the proliferative diseases described herein.

The term "individual" is a mammal, including humans. An individual includes, human, bovine, horse, feline, canine, rodent, or primate. In some embodiments, the individual is human. The individual (such as human) may have advanced disease or lesser extent of disease, such as low tumor burden. In some embodiments, the individual is at an early stage of a proliferative disease (such as cancer). In some embodiments, the individual is at an advanced stage of a proliferative disease (such as an advanced cancer). In some embodiments, the individual is HER2 positive. In some embodiments, the individual is HER2 negative.

The methods may be practiced in an adjuvant setting. "Adjuvant setting" refers to a clinical setting in which an individual has had a history of a proliferative disease, particularly cancer, and generally (but not necessarily) been responsive to therapy, which includes, surgery (such as surgical resection), radiotherapy, and chemotherapy. However, because of their history of the proliferative disease (such as cancer), these individuals are considered at risk of development of the disease. Treatment or administration in the "adjuvant setting" refers to a subsequent mode of treatment. The degree of risk (i.e., when an individual in the adjuvant setting is considered as "high risk" or "low risk") depends upon several factors, most usually the extent of disease when first treated. The methods may also be practiced in a neoadjuvant setting, i.e., the method may be carried out before the primary/definitive therapy. In some embodiments, the individual has previously been treated. In some embodiments, the individual has not previously been treated. In some embodiments, the treatment is a first line therapy.

### Proliferative diseases

The methods described herein are useful for treating proliferative diseases. In some embodiments, the proliferative disease is cancer. In some embodiments, the cancer is breast cancer, such as metastatic breast cancer. In some embodiments, the cancer is colon cancer. In some embodiments, the cancer is prostate cancer. In some embodiments, the cancer is a vascular tumor, which include, for example, medullary carcinoma of the thyroid, angiosarcoma, hemangioendothelioma, hemangioma and Kaposi's sarcoma.

Examples of cancers that may be treated using the compositions of the invention include, adenocortical carcinoma, agnogenic myeloid metaplasia, AIDS-related cancers (e.g., AIDS-related lymphoma), anal cancer, appendix cancer, astrocytoma (e.g., cerebellar and cerebral), basal cell carcinoma, bile duct cancer (e.g., extrahepatic), bladder cancer, bone cancer, (osteosarcoma and malignant fibrous histiocytoma), brain tumor (e.g., glioma, brain stem glioma, cerebellar or cerebral astrocytoma (e.g., pilocytic astrocytoma, diffuse astrocytoma, anaplastic (malignant) astrocytoma), malignant glioma, ependymoma, oligodenglioma, meningioma, craniopharyngioma, haemangioblastomas, medulloblastoma, supratentorial primitive neuroectodermal tumors, visual pathway and hypothalamic glioma, and glioblastoma), breast cancer, bronchial adenomas/carcinoids, carcinoid tumor (e.g., gastrointestinal carcinoid tumor), carcinoma of unknown primary, central nervous system lymphoma, cervical cancer, colon cancer, colorectal cancer, chronic myeloproliferative disorders, endometrial cancer (e.g., uterine cancer), ependymoma, esophageal cancer, Ewing's family of tumors, eye cancer (e.g., intraocular melanoma and retinoblastoma), gallbladder cancer, gastric (stomach) cancer, gastrointestinal carcinoid tumor, gastrointestinal stromal tumor (GIST), germ cell tumor, (e.g., extracranial, extragonadal, ovarian), gestational trophoblastic tumor, head and neck cancer, hepatocellular (liver) cancer (e.g., hepatic carcinoma and heptoma), hypopharyngeal cancer, islet cell carcinoma (endocrine pancreas), laryngeal cancer, laryngeal cancer, leukemia, lip and oral cavity cancer, oral cancer, liver cancer, lung cancer (e.g., small cell lung cancer, non-small cell lung cancer, adenocarcinoma of the lung, and squamous carcinoma of the lung), lymphoid neoplasm (e.g., lymphoma), medulloblastoma, melanoma, mesothelioma, metastatic squamous neck cancer, mouth cancer, multiple endocrine neoplasia syndrome, myelodysplastic syndromes, myelodysplastic/myeloproliferative diseases, nasal cavity and paranasal sinus cancer, nasopharyngeal cancer, neuroblastoma, neuroendocrine cancer, oropharyngeal cancer, ovarian cancer (e.g., ovarian epithelial cancer, ovarian germ cell tumor, ovarian low malignant potential tumor), pancreatic cancer, parathyroid cancer, penile cancer, cancer of the peritoneal, pharyngeal cancer, pheochromocytoma, pineoblastoma and supratentorial primitive neuroectodermal tumors, pituitary tumor, pleuropulmonary blastoma, lymphoma, primary central nervous system lymphoma (microglioma), pulmonary lymphangiomyomatosis, rectal cancer, renal cancer, renal pelvis and ureter cancer (transitional cell cancer), rhabdomyosarcoma, salivary gland cancer, skin cancer (e.g., non-melanoma (e.g., squamous cell carcinoma), melanoma, and Merkel cell carcinoma), small intestine cancer, squamous cell cancer, testicular cancer, throat cancer, thymoma and thymic carcinoma, thyroid cancer, tuberous sclerosis, urethral cancer, vaginal cancer, vulvar cancer, Wilms' tumor, and post-transplant lymphoproliferative disorder (PTLD), abnormal vascular proliferation associated with phakomatoses, edema (such as that associated with brain tumors), and Meigs' syndrome.

In some embodiments, the cancer is a solid tumor (such as advanced solid tumor). Solid tumor includes, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, Kaposi's sarcoma, soft tissue sarcoma, uterine sacronomasynovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma (including for example adenocarcinoma, clear cell renal cell carcinoma, papillary renal cell carcinoma, chromophobe renal cell carcinoma, collecting duct renal cell carcinoma, granular renal cell carcinoma, mixed granular renal cell carcinoma, renal angiomyolipomas, or spindle renal cell carcinoma.), hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma.

In some embodiments the lymphoid neoplasm (*e.g*., lymphoma) is a B-cell neoplasm. Examples of B-cell neoplasms included, precursor B-cell neoplasms (*e.g*., precursor B-lymphoblastic leukemia/lymphoma) and peripheral B-cell neoplasms (*e.g*., B-cell chronic lymphocytic leukemia/prolymphocytic leukemia/small lymphocytic lymphoma (small lymphocytic (SL) NHL), lymphoplasmacytoid lymphoma/immunocytoma, mantel cell lymphoma, follicle center lymphoma, follicular lymphoma (*e.g*., cytologic grades: I (small cell), II (mixed small and large cell), III (large cell) and/or subtype: diffuse and predominantly small cell type), low grade/follicular non-Hodgkin's lymphoma (NHL), intermediate grade/follicular NHL, marginal zone B-cell lymphoma (*e.g*., extranodal (*e.g*., MALT-type +/- monocytoid B cells) and/or Nodal (*e.g*., +/- monocytoid B cells)), splenic marginal zone lymphoma (*e.g*., +/- villous lymphocytes), Hairy cell leukemia, plasmacytoma/plasma cell myeloma (*e.g*., myeloma and multiple myeloma), diffuse large B-cell lymphoma (*e.g*., primary mediastinal (thymic) B-cell lymphoma), intermediate grade diffuse NHL, Burkitt's lymphoma, High-grade B-cell lymphoma, Burkitt-like, high grade immunoblastic NHL, high grade lymphoblastic NHL, high grade small non-cleaved cell NHL, bulky disease NHL, AIDS-related lymphoma, and Waldenstrom's macroglobulinemia).

In some embodiments the lymphoid neoplasm (*e.g*., lymphoma) is a T-cell and/or putative NK-cell neoplasm. Examples of T-cell and/or putative NK-cell neoplasms include, precursor T-cell neoplasm (precursor T-lymphoblastic lymphoma/leukemia) and peripheral T-cell and NK-cell neoplasms (*e.g*., T-cell chronic lymphocytic leukemia/prolymphocytic leukemia, and large granular lymphocyte leukemia (LGL) *(e.g.,* T-cell type and/or NK-cell type), cutaneous T-cell lymphoma (*e.g*., mycosis fungoides/Sezary syndrome), primary T-cell lymphomas unspecified *(e.g.,* cytological categories *(e.g.,* medium-sized cell, mixed medium and large cell), large cell, lymphoepitheloid cell, subtype hepatosplenic γd T-cell lymphoma, and subcutaneous panniculitic T-cell lymphoma), angioimmunoblastic T-cell lymphoma (AILD), angiocentric lymphoma, intestinal T-cell lymphoma (*e.g*., +/- enteropathy associated), adult T-cell lymphoma/leukemia (ATL), anaplastic large cell lymphoma (ALCL) (*e.g*., CD30+, T- and null-cell types), anaplastic large-cell lymphoma, and Hodgkin's like).

In some embodiments the lymphoid neoplasm (*e.g*., lymphoma) is Hodgkin's disease. For example, the Hodgkin's disease may be lymphocyte predominance, nodular sclerosis, mixed cellularity, lymphocyte depletion, and/or lymphocyte-rich.

In some embodiments, the cancer is leukemia. In some embodiments, the leukemia is chronic leukemia. Examples of chronic leukemia include, chronic myelocytic I (granulocytic) leukemia, chronic myelogenous, and chronic lymphocytic leukemia (CLL). In some embodiments, the leukemia is acute leukemia. Examples of acute leukemia include, acute lymphoblastic leukemia (ALL), acute myeloid leukemia, acute lymphocytic leukemia, and acute myelocytic leukemia (e.g., myeloblastic, promyelocytic, myelomonocytic, monocytic, and erythroleukemia).

In some embodiments, the cancer is liquid tumor or plasmacytoma. Plasmacytoma includes, myeloma. Myeloma includes, an extramedullary plasmacytoma, a solitary myeloma, and multiple myeloma. In some embodiments, the plasmacytoma is multiple myeloma.

In some embodiments, the cancer is multiple myeloma. Examples of multiple myeloma include, IgG multiple myeloma, IgA multiple myeloma, IgD multiple myeloma, IgE multiple myeloma, and nonsecretory multiple myeloma. In some embodiments, the multiple myeloma is IgG multiple myeloma. In some embodiments, the multiple myeloma is IgA multiple myeloma. In some embodiments, the multiple myeloma is a smoldering or indolent multiple myeloma. In some embodiments, the multiple myeloma is progressive multiple myeloma. In some embodiments, multiple myeloma may be resistant to a drug, such as, bortezomib, dexamethasone (Dex-), doxorubicin (Dox-), and melphalan (LR).

In some embodiments, the cancer is selected from the group consisting of phosphorylated-Akt positive advanced solid tumors, non-small cell lung cancer, sarcoma, Waldenstrom's macroglobulinemia, malignant melanoma, sarcoma, refractory and relapsed leukemia, Androgen-independent prostate cancer, advanced pancreatic cancer, recurrent, hormone sensitive prostate cancer, metastatic HNSCC, metastatic breast cancer, multiple myeloma, colorectal cancer, ovarian cancer, head and neck cancer, GIST, and relapsed epithelial ovarian cancer.

### Colchicine or thiocolchicine dimers

The methods described herein comprise administration of compositions comprising a colchicine or thiocolchicine dimer. "Colchicine or thiocolchicine dimer" used herein refers to a compound containing two (same or different) subunits of colchicine, thiocolchicine, or derivatives thereof. "Derivatives" of colchicine or thiocolchicine include, compounds that are structurally similar to colchicine or thiocolchicine or are in the same general chemical class as colchicine and thiocolchicine. Generally, the derivative or analog of colchicine or thiocolchicine retains similar biological, pharmacological, chemical and/or physical properties (including, for example, functionality) of colchicine or thiocolchicine. In some embodiments, the colchicine or thiocolchicine dimer comprises at least one thiocolchicine subunit. In some embodiments, the colchicine or thiocolchicine dimer comprises two thiocolchicine subunits (hereinafter referred to as "thiocolchicine dimer"). In some embodiments, the colchicine or thiocolchicine dimer comprises two colchicine subunits (herein after referred to as "colchicine dimer").

In some embodiments, the colchicine or thiocolchicine dimer is a compound of formula (I): 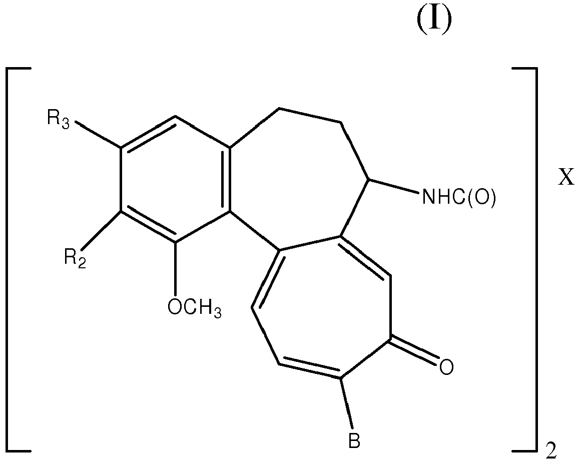 wherein the B in each subunit is either a methoxy or a methylthio group, R₂ is methoxy, hydroxyl, or methylenedioxy when taken together with R₃, R₃ is methoxy, hydroxyl, or methylenedioxy when taken together with R₂, and X is a linking group.

A wide variety of cross-linking groups can be used to introduce the linking group X. One of skill in the art will recognize that the colchine or thiocolchicine monomer components of the dimer have a single reactive amino group; should any other reactive (nucleophilic) groups be present on the intermediates, they can be readily protected using groups well-known in the art. For examples of protecting groups, see, for example, Greene, T.W., and P. G. M. Wuts, Protective Groups in Organic Synthesis, 3rd edition, Wiley: New York, 1999. Thus, a wide variety of cross-linking groups reactive with the amine functionality can be employed.

In some embodiments, the linking group X comprises at least one carbon atom. For example, the commercially available (Sigma-Aldrich) reagent malonyl chloride, Cl-C(O)-CH₂-C(O)-Cl, can be used to form a colchicine dimer where the X group is -CH₂-. Similarly, other diacyl chlorides of varying lengths can be used to form X groups of desired length. For example, in formula (II), when n = 8 and Y is CH₂, the commercially available (Sigma-Aldrich) reagent dodecanedioyl dichloride, Cl-C(O)-(CH₂)₁₀-C(O)-Cl, can be used to synthesize the dimer where the X group is -(CH₂)₁₀-. For groups where Y is NH and n = 1, the reagent 3-isocyanatopropanoyl chloride (Organic Syntheses, Coll. Vol. 6, p.715 (1988); Vol. 59, p.195 (1979)) can be used to synthesize the linking group X when X is -NH-CH₂CH₂-. Other well-known cross-linking reagents can be used to generate the X linker. One of skill in the art is directed to Wong, Shan S., Chemistry of Protein Conjugation and Cross Linking, CRC Press: Boca Raton, 1991, in particular, Chapter 2, Section IV(B)., pp. 33-38, directed towards amino-group reactive agents; Chapter 4, Section II, pp. 75-103, directed towards amino-group reactive cross-linkers; and Chapter 7, pp. 209-220, directed toward procedures and analysis for cross-linking reactions for reagents and procedures suitable for cross-linking amino-containing compounds.

In some embodiments, the colchicine or thiocolchicine dimer is a compound of the formula (II): 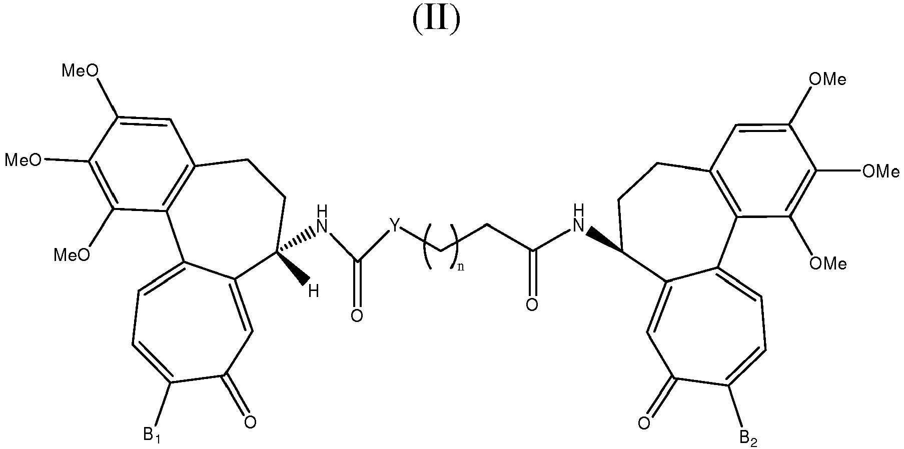 wherein B₁ is a methoxy or a methylthio group, B₂ is a methoxy or a methylthio group, n is an integer from 0 to 8, Y is a CH₂ group or, when n is 1, can also be a group of formula NH.

In some embodiments, n is any of (and in some embodiments selected from the group consisting of) 0, 1, 2, 3, 4, 5, 6, 7, or 8. In some embodiments, n is 1. In some embodiments, n is 1 and Y is NH. In some embodiments, n is 2.

In some embodiments, both B₁ and B₂ are methoxy groups. In some embodiments, both B₁ and B₂ are methylthio groups. In some embodiments, B₁ is methoxy group and B₂ is methylthio group. In some embodiments, B₁ is methylthio group and B₂ is methoxy group. In some embodiments, the colchicine or thiocolchicine dimer is any of (and in some embodiments selected from the group consisting of): IDN5404, IDN5676, IDN5800, and IDN5801.

In some embodiments, the compound is thiocolchicine dimer IDN5404. IDN5404 is a compound of formula (III):

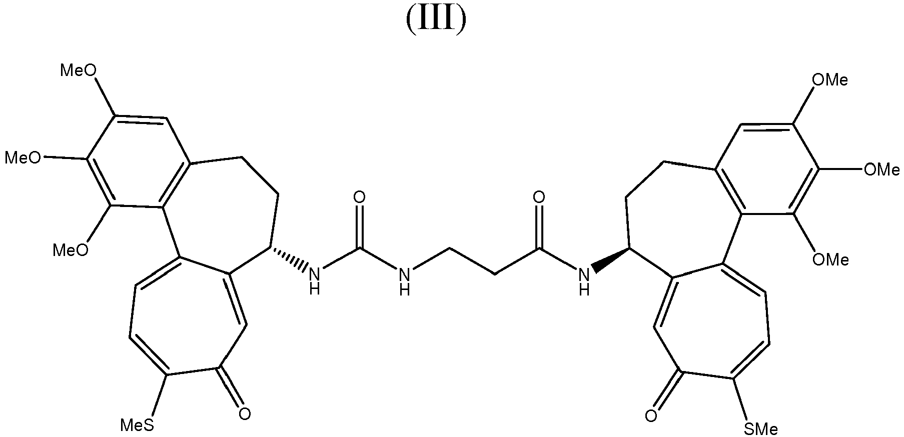

In some embodiments, the compound is thiocolchicine dimer IDN5676. IDN5676 is a compound of formula (IV):

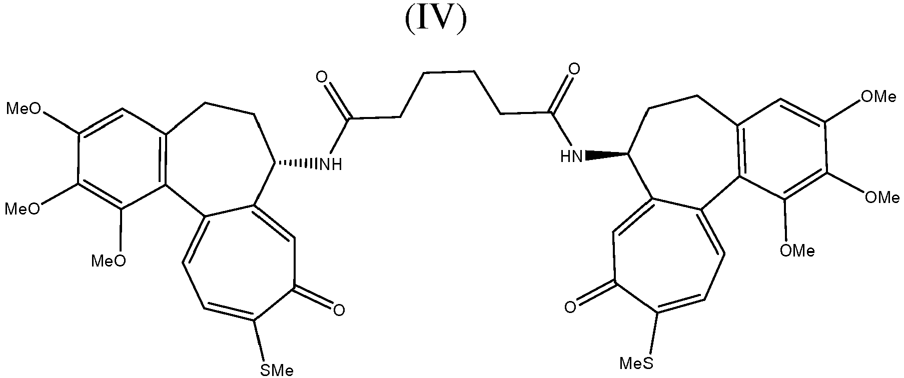

### Anti-VEGF antibodies

The methods described herein comprise administration of an anti-VEGF antibody. In some embodiments, the anti-VEGF antibody is a monoclonal antibody (such as full length monoclonal antibody). As used herein, "antibody" encompasses polyclonal and monoclonal antibodies, CDR-grafted antibodies, human antibodies, humanized antibodies, hybrid antibodies, altered antibodies, F(AB)'2 fragments, F(AB) molecules, Fv fragments, single domain antibodies, and chimeric antibodies. In some embodiments, the anti-VEGF antibody is a polyclonal antibody. In some embodiments, the anti-VEGF antibody is a chimeric antibody. In some embodiments, the antibody is a human antibody. In some embodiments, the anti-VEGF antibody is a humanized antibody. In some embodiments, the anti-VEGF antibody is a multispecific antibody (e.g., bispecific antibody). In some embodiments, the anti-VEGF antibody is a single chain Fv. In some embodiments, the anti-VEGF antibody is an antibody fragment (such as an Fab fragment).

In some embodiments, the anti-VEGF antibody is bevacizumab (or Avastin), or fragments thereof, e.g. Lucentis™ (also reviewed as rhuFAb V2 or AMD-Fab; ranibizumab, Genentech). In some embodiments, the anti-VEGF antibody bevacizumab. See US Pat. Nos. 6,054,297, 7,227,004; 6,884,879; 7.060,269; 7,169,901, and 7,297,334.

In some embodiments, the anti-VEGF antibody has the same or similar activity as bevacizumab. In some embodiments, the anti-VEGF antibody binds to the same or similar region or epitope as the bevacizumab or a fragment thereof. In some embodiments, the anti-VEGF antibody competes with the binding of bevacizumab or a fragment thereof to VEGF. In some embodiments, the anti-VEGF antibody is bioequivalent to bevacizumab or a fragment thereof. In some embodiments, the anti-VEGF antibody is biosimilar to bevacizumab or a fragment thereof. In some embodiments, the anti-VEGF antibody is a variant or derivative of bevacizumab, including functional fragments, derivatives, or antibody conjugates.

In some embodiments, the anti-VEGF antibody comprises a heavy chain variable region of bevacizumab. In some embodiments, the anti-VEGF antibody comprises a light chain variable region of bevacizumab. In some embodiments, the anti-VEGF antibody comprises a heavy chain variable region and a light chain variable region of bevacizumab. In some embodiments, the anti-VEGF antibody comprises a heavy chain variable region that is at least about 90%, including for example any one of 91%, 92%, 93%, 94%, 95%, 96%, 97%, 98%, or 99% homologous to the heavy chain variable region of bevacizumab. In some embodiments, the anti-VEGF antibody comprises a light chain variable region that is at least about 90%, including for example any one of 91%, 92%, 93%, 94%, 95%, 96%, 97%, 98%, or 99% homologous to the light chain variable region of bevacizumab. In some embodiments, the anti-VEGF antibody comprises a heavy chain variable region that is at least about 90%, including for example any one of 91%, 92%, 93%, 94%, 95%, 96%, 97%, 98%, or 99% homologous to the heavy chain variable region of bevacizumab and a light chain variable region that is at least about 90%, including for example any one of 91%, 92%, 93%, 94%, 95%, 96%, 97%, 98%, or 99% homologous to the light chain variable region of bevacizumab.

In some embodiments, the anti-VEGF antibody comprises a heavy chain variable region comprising CDR1, CDR2, and CDR3 of bevacizumab. In some embodiments, the anti-VEGF antibody comprises alight chain variable region comprising CDR1, CDR2, and CDR3 of bevacizumab. In some embodiments, the anti-VEGF antibody comprises a heavy chain variable region comprising CDR1, CDR2, and CDR3 of bevacizumab and the alight chain variable region comprising CDR1, CDR2, and CDR3 of bevacizumab. In some embodiments, the anti-VEGF antibody comprises a heavy chain variable region comprising CDR1, CDR2, and CDR3, wherein each of the CDR is at least about 90%, including for example any one of 91%, 92%, 93%, 94%, 95%, 96%, 97%, 98%, or 99% homologous to the corresponding heavy chain CDRs in bevacizumab. In some embodiments, the anti-VEGF antibody comprises a light chain variable region comprising CDR1, CDR2, and CDR3, wherein each of the CDR is at least about 90%, including for example any one of 91%, 92%, 93%, 94%, 95%, 96%, 97%, 98%, or 99% homologous to the corresponding light chain CDRs in bevacizumab. In some embodiments, the anti-VEGF antibody comprises a heavy chain variable region comprising CDR1, CDR2, and CDR3, wherein each of the CDR is at least about 90%, including for example any one of 91%, 92%, 93%, 94%, 95%, 96%, 97%, 98%, or 99% homologous to the corresponding heavy chain CDRs in bevacizumab, and a light chain variable region comprising CDR1, CDR2, and CDR3, wherein each of the CDR is at least about 90%, including for example any one of 91%, 92%, 93%, 94%, 95%, 96%, 97%, 98%, or 99% homologous to the corresponding light chain CDRs in bevacizumab.

In some embodiments, the anti-VEGF antibody comprises a heavy chain variable region comprising the heavy chain CDR3 of bevacizumab. In some embodiments, the anti-VEGF antibody comprises a light chain variable region comprising the light chain CDR3 of bevacizumab. In some embodiments, the anti-VEGF antibody comprises a heavy chain variable region comprising the heavy chain CDR3 of bevacizumab and the light chain variable region comprising the light chain CDR3 of bevacizumab. In some embodiments, the anti-VEGF antibody comprises a heavy chain variable region comprising a CDR3 that is at least about 90%, including for example any one of 91%, 92%, 93%, 94%, 95%, 96%, 97%, 98%, or 99% homologous to the heavy chain CDR3 in bevacizumab. In some embodiments, the anti-VEGF antibody comprises a light chain variable region comprising a CDR3 that is at least about 90%, including for example any one of 91%, 92%, 93%, 94%, 95%, 96%, 97%, 98%, or 99% homologous to the light chain CDR3 in bevacizumab. In some embodiments, the anti-VEGF antibody comprises a heavy chain variable region comprising a CDR3 that is at least about 90%, including for example any one of 91%, 92%, 93%, 94%, 95%, 96%, 97%, 98%, or 99% homologous to the heavy chain CDR3 in bevacizumab and a light chain variable region comprising a CDR3 that is at least about 90%, including for example any one of 91%, 92%, 93%, 94%, 95%, 96%, 97%, 98%, or 99% homologous to the light chain CDR3 in bevacizumab.

### Modes of administration

The colchicine or thiocolchicine dimer, the anti-VEGF antibody, and the taxane described herein can be administered simultaneously (i.e., simultaneous administration) and/or sequentially (i.e., sequential administration) with respect to each other.

In some embodiments, the colchicine or thiocolchicine dimer and the taxane are administered simultaneously. In some embodiments, the colchicine or thiocolchicine dimer and the anti-VEGF antibody are administered simultaneously. The term "simultaneous administration," as used herein, means that the nanoparticle composition and the other agent are administered with a time separation of no more than about 15 minute(s), such as no more than about any of 10, 5, or 1 minutes. When the drugs are administered simultaneously, they may be contained in the same composition or in separate compositions.

In some embodiments, the colchicine or thiocolchicine dimer and the taxane are administered sequentially. In some embodiments, the colchicine or thiocolchicine dimer and the anti-VEGF antibody are administered sequentially. The term "sequential administration" as used herein means that the drugs are administered with a time separation of more than about 15 minutes, such as more than about any of 20, 30, 40, 50, 60 or more minutes. The drugs are contained in separate compositions, which may be contained in the same or different packages. In some embodiments, the colchicine or thiocolchicine dimer is administered no greater than about 24 hours prior to the administration of the anti-VEGF antibody. In some embodiments, the colchicine or thiocolchicine dimer is administered no greater than about 24 hours prior to the administration of the taxane.

In some embodiments, the administration of the colchicine or thiocolchicine dimer and the anti-VEGF antibody (or the taxane) are concurrent, i.e., the administration period of the drugs overlap with each other.

The dosing frequency of the colchicine or thiocolchicine dimer and the anti-VEGF antibody (or the taxane) may be adjusted over the course of the treatment, based on the judgment of the administering physician. When administered separately, the colchicine or thiocolchicine dimer and the anti-VEGF antibody (or the taxane) can be administered at different dosing frequency or intervals.

Exemplary dosing frequencies for drugs described herein include, weekly without break; weekly, three out of four weeks; once every three weeks; once every two weeks; weekly, two out of three weeks. In some embodiments, the drug is administered about once every 2 weeks, once every 3 weeks, once every 4 weeks, once every 6 weeks, or once every 8 weeks. In some embodiments, the composition is administered at least about any of 1x, 2x, 3x, 4x, 5x, 6x, or 7x (i.e., daily) a week. In some embodiments, the intervals between each administration are less than about any of 6 months, 3 months, 1 month, 20 days, 15, days, 12 days, 10 days, 9 days, 8 days, 7 days, 6 days, 5 days, 4 days, 3 days, 2 days, or 1 day. In some embodiments, the intervals between each administration are more than about any of 1 month, 2 months, 3 months, 4 months, 5 months, 6 months, 8 months, or 12 months. In some embodiments, there is no break in the dosing schedule. In some embodiments, the interval between each administration is no more than about a week.

The colchicine or thiocolchicine dimer and the anti-VEGF antibody (or the taxane) can be administered using the same route of administration or different routes of administration. In some embodiments (for both simultaneous and sequential administrations), the colchicine or thiocolchicine dimer and the anti-VEGF antibody (or the taxane) are administered at a predetermined ratio. For example, in some embodiments, the ratio by weight of the colchicine or thiocolchicine dimer and the anti-VEGF antibody (or the taxane) is about 1 to 1. In some embodiments, the weight ratio may be between about 0.001 to about 1 and about 1000 to about 1, or between about 0.01 to about 1 and 100 to about 1. In some embodiments, the ratio is less than about any of 100:1, 50:1, 30:1, 10:1, 9:1, 8:1, 7:1, 6:1, 5:1, 4:1, 3:1, 2:1, and 1:1 In some embodiments, the ratio by weight is more than about any of 1:1, 2:1, 3:1, 4:1, 5:1, 6:1, 7:1, 8:1, 9:1, 30:1, 50:1, 100:1. Other ratios are contemplated.

The doses required for the colchicine or thiocolchicine dimer and the anti-VEGF antibody (and/or the taxane) may (but not necessarily) be lower than what is normally required when each agent is administered alone. Thus, in some embodiments, a subtherapeutic amount of the drugs are administered. "Subtherapeutic amount" or "subtherapeutic level" refers to an amount that is less than therapeutic amount, that is, less than the amount normally used when the drugs are administered alone. The reduction may be reflected in terms of the amount administered at a given administration and/or the amount administered over a given period of time (reduced frequency).

In some embodiments, enough other agent is administered so as to allow reduction of the normal dose of the drug (such as the colchicine or thiocolchicine dimer) required to effect the same degree of treatment by at least about any of 5%, 10%, 20%, 30%, 50%, 60%, 70%, 80%, 90%, or more. In some embodiments, enough drug is administered so as to allow reduction of the normal dose of the other agent required to effect the same degree of treatment by at least about any of 5%, 10%, 20%, 30%, 50%, 60%, 70%, 80%, 90%, or more.

In some embodiments, the dose of the colchicine or thiocolchicine dimer and/or the anti-VEGF antibody (and/or taxane) is reduced as compared to the corresponding normal dose of each when administered alone. In some embodiments, the colchicine or thiocolchicine dimer and/or the anti-VEGF antibody (and/or taxane) is administered at a subtherapeutic, i.e., reduced, level. In some embodiments, the dose of the colchicine or thiocolchicine dimer and/or the anti-VEGF antibody (and/or taxane) is substantially less than the established maximum toxic dose (MTD). For example, the dose can be less than about 50%, 40%, 30%, 20%, or 10% of the MTD.

In some embodiments, the doses of the colchicine or thiocolchicine dimer and/or the anti-VEGF antibody (and/or taxane) are higher than what is normally required when each agent is administered alone. For example, in some embodiments, the doses of the colchicine or thiocolchicine dimer and/or the anti-VEGF antibody (and/or taxane) are substantially higher than the established maximum toxic dose (MTD). For example, the dose of the doses of the colchicine or thiocolchicine dimer and/or the anti-VEGF antibody (and/or taxane) is more than about 50%, 40%, 30%, 20%, or 10% of the MTD of the agent when administered alone.

In some embodiments, the amount of the colchicine or thiocolchicine dimer is included in any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some embodiments, the amount of colchicine or thiocolchicine dimer in the effective amount of the composition (e.g., a unit dosage form) is in the range of about 5 mg to about 500 mg, such as about 30 mg to about 300 mg or about 50 mg to about 200 mg.

For example, the colchicine or thiocolchicine dimer can be administered at a dose of about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). For example, in some embodiments, the colchicine or thiocolchicine dimer is administered at about 20-100 mg/kg (including for example 30 mg/kg, 40 mg/kg, 50 mg/kg, 60 mg/kg, 70 mg/kg, 80 mg/kg), three times a week.

Exemplary effective amounts of the colchicine or thiocolchicine dimer include, about any of 25 mg/m2, 30 mg/m2, 50 mg/m2, 60 mg/m2, 75 mg/m2, 80 mg/m2, 90 mg/m2, 100 mg/m2, 120 mg/m2, 125 mg/m2, 150 mg/m2, 160 mg/m2, 175 mg/m2, 180 mg/m2, 200 mg/m2, 210 mg/m2, 220 mg/m2, 250 mg/m2, 260 mg/m2, 300 mg/m2, 350 mg/m2, 400 mg/m2, 500 mg/m2, 540 mg/m2, 750 mg/m2, 1000 mg/m2, or 1080 mg/m2. In various embodiments, the composition includes less than about any of 350 mg/m2, 300 mg/m2, 250 mg/m2, 200 mg/m2, 150 mg/m2, 120 mg/m2, 100 mg/m2, 90 mg/m2, 50 mg/m2, or 30 mg/m2 of the colchicine or thiocolchicine dimer. In some embodiments, the amount of the colchicine or thiocolchicine dimer per administration is less than about any of 25 mg/m2, 22 mg/m2, 20 mg/m2, 18 mg/m2, 15 mg/m2, 14 mg/m2, 13 mg/m2, 12 mg/m2, 11 mg/m2, 10 mg/m2, 9 mg/m2, 8 mg/m2, 7 mg/m2, 6 mg/m2, 5 mg/m2, 4 mg/m2, 3 mg/m2, 2 mg/m2, or 1 mg/m2. In some embodiments, the effective amount of the colchicine or thiocolchicine dimer is included in any of the following ranges: about 1 to about 5 mg/m2, about 5 to about 10 mg/m2, about 10 to about 25 mg/m2, about 25 to about 50 mg/m2, about 50 to about 75 mg/m2, about 75 to about 100 mg/m2, about 100 to about 125 mg/m2, about 125 to about 150 mg/m2, about 150 to about 175 mg/m2, about 175 to about 200 mg/m2, about 200 to about 225 mg/m2, about 225 to about 250 mg/m2, about 250 to about 300 mg/m2, about 300 to about 350 mg/m2, or about 350 to about 400 mg/m2. Preferably, the effective amount of a taxane (e.g., paclitaxel) in the composition is about 5 to about 300 mg/m2, such as about 20 to about 150 mg/m2, or about 30 to about 100 mg/m2.

In some embodiments of any of the above aspects, the effective amount of the colchicine or thiocolchicine dimer includes at least about any of 1 mg/kg, 2.5 mg/kg, 3.5 mg/kg, 5 mg/kg, 6.5 mg/kg, 7.5 mg/kg, 10 mg/kg, 15 mg/kg, 20 mg/kg, 30 mg/kg, Or 40 mg/kg. In various embodiments, the effective amount of the colchicine or thiocolchicine dimer in the composition includes less than about any of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg of the colchicine or thiocolchicine dimer. In some embodiments, the effective amount of the colchicine or thiocolchicine dimer is about any of 1-350 mg/m2, 5-100 mg/m2, or 10-30 mg/m2.

Suitable dosages for anti-VEGF antibody include, for example, about 1 mg/kg to about 80 mg/kg, including for example about 1 mg/kg to about 50 mg/kg, such as about 1 mg/kg to about 15 mg/kg (such as about any of 2, 4, 6, 8, 10, or 12 mg/kg). In some embodiments, the dosage of the anti-VEGF antibody is about 40 mg/m2 to about 600 mg/m2, including for example about 100 mg/m2 to about 400 mg/m2 (such as about any of 100, 200, or 300 mg/m2).

Exemplary combinations of the amounts of colchicine or thiocolchicine dimer and the anti-VEGF antibody include, for example, about 1 mg/kg to about 50 mg/kg (such as about any of 2, 5, 10, or 15 mg/kg) colchicine or thiocolchicine dimer and about 1 mg/kg to about 20 mg/kg (such as about any of 2, 4, 6, 8, 10, 12, 14, 16, or 18 mg/kg) anti-VEGF antibody; about 3 mg/m2 to about 400 mg/m2 (such as about any of 6, 10, 15, 30, 45, 60, 100, 150, 200, or 300 mg/m2) colchicine or thiocolchicine dimer and 40 mg/m2 to about 600 mg/m2, including for example about 100 mg/m2 to about 400 mg/m2 (such as about any of 100, 200, or 300 mg/m2) anti-VEGF antibody; about 3 mg/m2 to about 300 mg/m2 (such as about any of 6, 10, 15, 30, 45, 60, 100, 150, 200, or 300 mg/m2) colchicine or thiocolchicine dimer and about 1 mg/kg to about 20 mg/kg (such as about any of 2, 4, 6, 8, 10, 12, 14, 16, or 18 mg/kg) anti-VEGF antibody. In some embodiments, the method comprises administering to an individual at least about 30 mg/m2 colchicine or thiocolchicine dimer and at least about any of 2, 4, 8, or 10 mg/kg anti-VEGF antibody. In some embodiments, the taxane is paclitaxel. In some embodiments, the anti-VEGF antibody is bevacizumab (such as Avastin®). In some embodiments, the taxane is paclitaxel and the anti-VEGF antibody is bevacizumab (such as Avastin®).

Other exemplary dosing regime for the combination therapy of the colchicine or thiocolchicine dimer and the anti-VEGF antibody includes administration of 30 mg/m2 -100 mg/m2 (such as 30-50 mg/m2) colchicine or thiocolchicine dimer at least once every three weeks (including for example once every 1 day, 2 days, 3 days, 4 days, 5 days, 6 days, 1 week, 2 weeks) concurrent with administration of 2 mg/kg -15 mg/kg (such as any of 4, 6, 8, 10 mg/kg or 15 mg/kg) anti-VEGF antibody every two weeks or more frequently (for example every week, twice every week, or three times a week). In some embodiments, the anti-VEGF antibody is bevacizumab (such as Avastin®). In some embodiments, the colchicine or thiocolchicine dimer is IDN-5404 and the anti-VEGF antibody is bevacizumab (such as Avastin®).

In some embodiments, the effective amount of the colchicine or thiocolchicine dimer is between about 30 mg/m2 to about 100 mg/m2 and the effective amount of anti-VEGF antibody is greater than about 1 mg/kg to less than about 10 mg/kg.

In some embodiments, when taxane is administered, the effective amount of the anti-VEGF antibody is an amount effective to suppress taxane-mediated induction of VEGF in vivo. In some embodiments, the taxane-mediated induction of VEGF in vivo is taxane-mediated induction of VEGF-A.

The term "amount effective to suppress taxane-mediated induction of VEGF in vivo," as used herein, refers to and includes both complete (including substantially complete) and/or partial suppression. Methods indicating such suppression are known in the art and described herein, although it is understood that when administering to an individual patient based on established medical practice on the basis of clinical trials, such measurements need not be given in an individual. In some embodiments, the term "amount effective to suppress taxane-mediated induction of VEGF," as used herein, refers to substantially complete prevention of VEGF expression and/or activity or reduction in the amount VEGF (such as VEGF-A) expression and/or activity in cells, tissues or fluids in vivo upon administration of a formulation containing a taxane. In some embodiments, the reduction in the amount VEGF expression and/or activity in cells, tissues or fluids in vivo upon administration of a formulation containing a taxane are by at least about any of 10%, 20%, 30%, 40%, 50%, 60%, 70%, 80%, 90%, 95%, or 100%. In some embodiments, the suppression of taxane induction can be observed qualitatively and/or quantitatively by methods known in the art and described herein.

In some embodiments, the amount of a taxane (e.g., paclitaxel) is included in any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some embodiments, the amount of a taxane (e.g., paclitaxel) or derivative thereof in the effective amount of the composition (e.g., a unit dosage form) is in the range of about 5 mg to about 500 mg, such as about 30 mg to about 300 mg or about 50 mg to about 200 mg. In some embodiments, the concentration of the taxane (e.g., paclitaxel) in the composition is dilute (about 0.1 mg/ml) or concentrated (about 100 mg/ml), including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg/ml. In some embodiments, the concentration of the taxane (e.g., paclitaxel) is at least about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml.

Exemplary effective amounts of a taxane (e.g., paclitaxel) in the nanoparticle composition include, about any of 25 mg/m2, 30 mg/m2, 50 mg/m2, 60 mg/m2, 75 mg/m2, 80 mg/m2, 90 mg/m2, 100 mg/m2, 120 mg/m2, 125 mg/m2, 150 mg/m2, 160 mg/m2, 175 mg/m2, 180 mg/m2, 200 mg/m2, 210 mg/m2, 220 mg/m2, 250 mg/m2, 260 mg/m2, 300 mg/m2, 350 mg/m2, 400 mg/m2, 500 mg/m2, 540 mg/m2, 750 mg/m2, 1000 mg/m2, or 1080 mg/m2 of a taxane (e.g., paclitaxel). In various embodiments, the composition includes less than about any of 350 mg/m2, 300 mg/m2, 250 mg/m2, 200 mg/m2, 150 mg/m2, 120 mg/m2, 100 mg/m2, 90 mg/m2, 50 mg/m2, or 30 mg/m2 of a taxane (e.g., paclitaxel). In some embodiments, the amount of the taxane (e.g., paclitaxel) per administration is less than about any of 25 mg/m2, 22 mg/m2, 20 mg/m2, 18 mg/m2, 15 mg/m2, 14 mg/m2, 13 mg/m2, 12 mg/m2, 11 mg/m2, 10 mg/m2, 9 mg/m2, 8 mg/m2, 7 mg/m2, 6 mg/m2, 5 mg/m2, 4 mg/m2, 3 mg/m2, 2 mg/m2, or 1 mg/m2. In some embodiments, the effective amount of a taxane (e.g., paclitaxel) in the composition is included in any of the following ranges: about 1 to about 5 mg/m2, about 5 to about 10 mg/m2, about 10 to about 25 mg/m2, about 25 to about 50 mg/m2, about 50 to about 75 mg/m2, about 75 to about 100 mg/m2, about 100 to about 125 mg/m2, about 125 to about 150 mg/m2, about 150 to about 175 mg/m2, about 175 to about 200 mg/m2, about 200 to about 225 mg/m2, about 225 to about 250 mg/m2, about 250 to about 300 mg/m2, about 300 to about 350 mg/m2, or about 350 to about 400 mg/m2. Preferably, the effective amount of a taxane (e.g., paclitaxel) in the composition is about 5 to about 300 mg/m2, such as about 100 to about 150 mg/m2, about 120 mg/m2, about 130 mg/m2, or about 140 mg/m2.

In some embodiments of any of the above aspects, the effective amount of a taxane (e.g., paclitaxel) in the composition includes at least about any of 1 mg/kg, 2.5 mg/kg, 3.5 mg/kg, 5 mg/kg, 6.5 mg/kg, 7.5 mg/kg, 10 mg/kg, 15 mg/kg, or 20 mg/kg. In various embodiments, the effective amount of a taxane (e.g., paclitaxel) in the composition includes less than about any of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg of a taxane (e.g., paclitaxel) .

Other exemplary dosing schedules for the administration of the taxane (such as nanoparticle composition of taxane, including paclitaxel/albumin nanoparticle composition, for example Abraxane®) include, 100 mg/m2, weekly, without break; 75 mg/m2 weekly, 3 out of four weeks; 100 mg/m2, weekly, 3 out of 4 weeks; 125 mg/m2, weekly, 3 out of 4 weeks; 125 mg/m2, weekly, 2 out of 3 weeks; 130 mg/m2, weekly, without break; 175 mg/m2, once every 2 weeks; 260 mg/m2, once every 2 weeks; 260 mg/m2, once every 3 weeks; 180-300 mg/m2, every three weeks; 60-175 mg/m2, weekly, without break. In addition, the taxane (alone or in combination therapy) can be administered by following a metronomic dosing regime described herein.

Suitable combinations of the amounts of taxane and the anti-VEGF antibody include, for example, about 1 mg/kg to about 20 mg/kg (such as about any of 2, 5, 10, or 15 mg/kg) taxane and about 1 mg/kg to about 20 mg/kg (such as about any of 2, 4, 6, 8, 10, 12, 14, 16, or 18 mg/kg) anti-VEGF antibody; about 3 mg/m2 to about 400 mg/m2 (such as about any of 6, 10, 15, 30, 45, 60, 100, 150, 200, or 300 mg/m2) taxane and 40 mg/m2 to about 600 mg/m2, including for example about 100 mg/m2 to about 400 mg/m2 (such as about any of 100, 200, or 300 mg/m2) anti-VEGF antibody; about 3 mg/m2 to about 300 mg/m2 (such as about any of 6, 10, 15, 30, 45, 60, 100, 150, 200, or 300 mg/m2) taxane and about 1 mg/kg to about 20 mg/kg (such as about any of 2, 4, 6, 8, 10, 12, 14, 16, or 18 mg/kg) anti-VEGF antibody. In some embodiments, the method comprises administering to an individual at least about 200 mg/m2 taxane and at least about any of 2, 4, 8, or 10 mg/kg anti-VEGF antibody. In some embodiments, the taxane is paclitaxel. In some embodiments, the taxane is docetaxel. In some embodiments, the anti-VEGF antibody is bevacizumab (such as Avastin®). In some embodiments, the anti-VEGF antibody is bevacizumab (such as Avastin®) and the taxane is paclitaxel.

In some embodiments, the taxane and the anti-VEGF antibody are administered simultaneously to the individual. In some embodiments of the methods, the administration of the taxane and the anti-VEGF antibody are concurrent. One exemplary dosing regime for the combination therapy of taxane (such as paclitaxel) includes administration of 100 mg/m2 -300 mg/m2 (such as 200 mg/m2) taxane at least weekly (including for example every 1, 2, 3, 4, 5, or 6 days) concurrent with administration of 2 mg/kg -15 mg/kg (such as any of 4, 6, 8, 10 mg/kg or 15 mg/kg) anti-VEGF antibody every two weeks or more frequently (for example every week, twice every week, or three times a week).

In some embodiments, the taxane and the anti-VEGF antibody are administered sequentially to the individual. For example, in some embodiments, the taxane is administered for at least one (such as at least any of two, three, four, five, or six) cycles prior to the administration of the anti-VEGF antibody. This is then followed by the administration of an anti-VEGF antibody for at least once (such as twice) a week for at least about 3 (such as 4, 5, or 6) weeks. One exemplary dosing regime for the combination therapy of taxane composition (such as paclitaxel/albumin nanoparticle composition, for example Abraxane®) and anti-VEGF antibody (such as bevacizumab, for example Avastin®) includes administration of 10 mg/kg colchicine or thiocolchicine dimer daily for 5 days in two cycles separated by one week followed by administration of an anti-VEGF antibody at dosages of 2 mg/kg, 4 mg/kg, or 8 mg/kg twice a week for 6 weeks. In some embodiments, the taxane is paclitaxel. In some embodiments, the taxane is docetaxel. In some embodiments, the anti-VEGF antibody is bevacizumab (such as Avastin®). In some embodiments, the anti-VEGF antibody is bevacizumab (such as Avastin®) and the taxane is paclitaxel.

In some embodiments, the effective amount of taxane is between about 45 mg/m2 to about 350 mg/m2 and the effective amount of anti-VEGF antibody is between about 1 mg/kg to about 20 mg/kg. In some embodiments, the effective amount of taxane in the composition is between about 80 mg/m2 to about 150 mg/m2 and the effective amount of anti-VEGF antibody is between about 1 mg/kg to about 20 mg/kg. In some embodiments, the effective amount of taxane in the composition is about 100 mg/m2. In some embodiments, the taxane is administered weekly. In some embodiments, the effective amount of taxane in the composition is between about 170 mg/m2 to about 200 mg/m2 and the effective amount of anti-VEGF antibody is between about 1 mg/kg to about 20 mg/kg. In some embodiments, the effective amount of taxane in the composition is between about 200 mg/m2 to about 350 mg/m2 and the effective amount of anti-VEGF antibody is between about 1 mg/kg to about 20 mg/kg. In some embodiments, the taxane is administered every two weeks. In some embodiments, the effective amount of taxane in the composition is about 260 mg/m2. In some embodiments, the taxane is administered every three weeks. In some embodiments of any of the above methods, the effective amount of anti-VEGF antibody is greater than 1 mg/kg to less than 10 mg/kg or greater than 15 mg/kg to less than 20 mg/kg. In some embodiments, the effective amount of anti-VEGF antibody is between about 5 to about 10 mg/kg. In some embodiments of any of the above methods, the effective amount of anti-VEGF antibody is about 2 mg/kg, about 4 mg/kg, about 6 mg/kg, about 8 mg/kg, about 10 mg/kg, about 12 mg/kg, or about 15 mg/kg. In some embodiments, the effective amount of anti-VEGF antibody is about 10 mg/kg. In some embodiments, the effective amount of anti-VEGF antibody is about 15 mg/kg. In some embodiments, the anti-VEGF antibody is administered every two weeks or every three weeks. In some embodiments, the taxane is paclitaxel. In some embodiments, the taxane is docetaxel. In some embodiments, the anti-VEGF antibody is bevacizumab (such as Avastin®). In some embodiments, the anti-VEGF antibody is bevacizumab (such as Avastin®) and the taxane is paclitaxel.

In some embodiments, the effective amount of the anti-VEGF antibody is an amount effective to suppress taxane-mediated induction of VEGF in vivo. In some embodiments, the taxane-mediated induction of VEGF in vivo is taxane-mediated induction of VEGF-A. In some embodiments, the effective amount of taxane in the nanoparticle composition is between about 45 mg/m2 to about 350 mg/m2 and the effective amount of anti-VEGF antibody is between about 1 mg/kg to about 20 mg/kg. In some embodiments, the effective amount of taxane in the nanoparticle composition is between about 80 mg/m2 to about 150 mg/m2 and the effective amount of anti-VEGF antibody is between about 1 mg/kg to about 20 mg/kg. In some embodiments, the effective of amount of taxane in the nanoparticle composition is about 100 mg/m2. In some embodiments, the colchicine or thiocolchicine dimer is administered weekly. In some embodiments, the effective amount of taxane in the nanoparticle composition is between about 170 mg/m2 to about 200 mg/m2 and the effective amount of anti-VEGF antibody is between about 1 mg/kg to about 20 mg/kg. In some embodiments, the colchicine or thiocolchicine dimer is administered every two weeks. In some embodiments, the effective amount of taxane is between about 200 mg/m2 to about 350 mg/m2 and the effective amount of anti-VEGF antibody is between about 1 mg/kg to about 20 mg/kg. In some embodiments, the effective amount of taxane is about 260 mg/m2. In some embodiments, taxane is administered every three weeks. In some embodiments, the anti-VEGF antibody is administered every 2 weeks or every 3 weeks. In some embodiments, the effective amount of anti-VEGF antibody is greater than 1 mg/kg to less than 10 mg/kg or greater than 15 mg/kg to less than 20 mg/kg. In some embodiments, the effective amount of anti-VEGF antibody is between about 5 mg/kg and about 10 mg/kg. In some embodiments, the effective amount of anti-VEGF antibody is about 2 mg/kg, about 4 mg/kg, about 6 mg/kg, about 8 mg/kg, about 10 mg/kg, about 12 mg/kg, or about 15 mg/kg. In some embodiments, the effective amount of anti-VEGF antibody is about 10 mg/kg. In some embodiments, the effective amount of anti-VEGF antibody is about 15 mg/kg.

In some embodiments, the taxane is in a nanoparticle form. In some embodiments, the taxane composition is Abraxane®. In some embodiments, the anti-VEGF antibody is bevacizumab (i.e., Avastin®). In some embodiments, the effective amount of the taxane composition (such as Abraxane®) is between about 45 mg/m2 to about 350 mg/m2 and the effective amount of bevacizumab is between about 1 mg/kg to about 20 mg/kg. In some embodiments, the effective amount of the taxane composition (such as Abraxane®) is between about 80 mg/m2 to about 150 mg/m2 and the effective amount of bevacizumab is between about 1 mg/kg to about 20 mg/kg. In some embodiments, the effective of amount of the taxane composition (such as Abraxane®) is about 100 mg/m2. In some embodiments, the taxane composition (such as Abraxane®) is administered weekly. In some embodiments, the effective amount of the taxane composition (such as Abraxane®) is between about 170 mg/m2 to about 200 mg/m2 and the effective amount of bevacizumab is between about 1 mg/kg to about 20 mg/kg. In some embodiments, the taxane composition (such as Abraxane®) is administered every two weeks. In some embodiments, the effective amount of the taxane composition (such as Abraxane®) is between about 200 mg/m2 to about 350 mg/m2 and the effective amount of bevacizumab is between about 1 mg/kg to about 20 mg/kg. In some embodiments, the effective amount of the taxane composition (such as Abraxane®) is about 260 mg/m2. In some embodiments, the taxane composition (such as Abraxane®) is administered every three weeks. In some embodiments, bevacizumab is administered every 2 weeks or every 3 weeks. In some embodiments of any of the above methods, the effective amount of bevacizumab is greater than 1 mg/kg to less than 10 mg/kg or greater than 15 mg/kg to less than 20 mg/kg. In some embodiments, the effective amount of bevacizumab is between about 5 mg/kg and about 10 mg/kg. In some embodiments, the effective amount of bevacizumab is about 2 mg/kg, about 4 mg/kg, about 6 mg/kg, about 8 mg/kg, about 10 mg/kg, about 12 mg/kg, or about 15 mg/kg. In some embodiments, the effective amount of bevacizumab is about 10 mg/kg. In some embodiments, the effective amount of bevacizumab is about 15 mg/kg.

In some embodiments, the effective amount of the taxane composition (such as Abraxane®) is between about 80 mg/m2 to about 150 mg/m2 and the effective amount of bevacizumab is about 10 mg/kg or about 15 mg/kg. In some embodiments, the effective amount of the taxane composition (such as Abraxane®) is about 100 mg/m2. In some embodiments, the taxane composition (such as Abraxane®) is administered weekly. In some embodiments, bevacizumab is administered every 2 weeks or every 3 weeks.

In some embodiments, the effective amount of the taxane composition (such as Abraxane®) is between about 200 mg/m2 to about 350 mg/m2 and the effective amount of bevacizumab is between about 5 mg/kg and about 15 mg/kg. In some embodiments, the effective amount of the taxane composition (such as Abraxane®) is about 260 mg/m2. In some embodiments, the taxane composition (such as Abraxane®) is administered weekly. In some embodiments, the effective amount of bevacizumab is between about 5 mg/kg and about 10 mg/kg. In some embodiments, the effective amount of bevacizumab is about 15 mg/kg. In some embodiments, bevacizumab is administered every 2 weeks or every 3 weeks.

In some embodiments, the anti-VEGF antibody is administered every 2 weeks or every 3 weeks. In some embodiments of any of the above methods, the effective amount of anti-VEGF antibody is greater than 1 mg/kg to less than 10 mg/kg or greater than 15 mg/kg to less than 20 mg/kg. In some embodiments, the effective amount of anti-VEGF antibody is between about 5 mg/kg and about 10 mg/kg. In some embodiments, the effective amount of anti-VEGF antibody is about 2 mg/kg, about 4 mg/kg, about 6 mg/kg, about 8 mg/kg, about 10 mg/kg, about 12 mg/kg, or about 15 mg/kg. In some embodiments, the effective amount of anti-VEGF antibody is about 10 mg/kg. In some embodiments, the effective amount of anti-VEGF antibody is about 15 mg/kg.

In some embodiments, the colchicine or thiocolchicine dimer, the anti-VEGF antibody, and the taxane are administered according to the dosing schedule listed in any of Tables 1-7.

The administration of the drugs can be extended over an extended period of time, such as from about a month up to about seven years. In some embodiments, the drug is administered over a period of at least about any of 2, 3, 4, 5, 6, 7, 8, 9, 10, 11, 12, 18, 24, 30, 36, 48, 60, 72, or 84 months.

In some embodiments, the individual is treated for at least about any of one, two, three, four, five, six, seven, eight, nine, or ten treatment cycles. The compositions described herein allow infusion of the composition to an individual over an infusion time that is shorter than about 24 hours. For example, in some embodiments, the composition is administered over an infusion period of less than about any of 24 hours, 12 hours, 8 hours, 5 hours, 3 hours, 2 hours, 1 hour, 30 minutes, 20 minutes, or 10 minutes. In some embodiments, the composition is administered over an infusion period of about 30 minutes.

The drugs described herein can be administered to an individual (such as human) via various routes, including, for example, intravenous, intra-arterial, intraperitoneal, intrapulmonary, oral, inhalation, intravesicular, intramuscular, intratracheal, subcutaneous, intraocular, intrathecal, transmucosal, and transdermal. In some embodiments, sustained continuous release formulation of the composition may be used. In one variation of the invention, nanoparticles (such as albumin nanoparticles) of the inventive compounds can be administered by any acceptable route including, but not limited to, orally, intramuscularly, transdermally, intravenously, through an inhaler or other air borne delivery systems and the like.

A combination of the administration configurations described herein can be used. The combination therapy methods described herein may be performed alone or in conjunction with another therapy, such as surgery, radiation, chemotherapy, immunotherapy, gene therapy, and the like. Additionally, a person having a greater risk of developing the proliferative disease may receive treatments to inhibit or and/or delay the development of the disease.

As will be understood by those of ordinary skill in the art, the appropriate doses of other agents will be approximately those already employed in clinical therapies wherein the other agent are administered alone or in combination with other agents. Variation in dosage will likely occur depending on the condition being treated. As described above, in some embodiments, the other agents may be administered at a reduced level.

### Nanoparticle compositions

The nanoparticle compositions described herein comprise nanoparticles comprising (in various embodiments consisting essentially of) a colchchicine or thiocolchicine (or a taxane) and a carrier protein (such as albumin). Nanoparticles of poorly water soluble drugs (such as taxane) have been disclosed in, for example, U.S. Pat. Nos. 5,916,596; 6,506,405; 6,749,868, and 6,537,579 and also in U.S. Pat. Pub. Nos. 2005/0004002 and 2007/0082838.

In some embodiments, the composition comprises nanoparticles with an average or mean diameter of no greater than about 1000 nanometers (nm), such as no greater than about any of 900, 800, 700, 600, 500, 400, 300, 200, and 100 nm. In some embodiments, the average or mean diameters of the nanoparticles is no greater than about 200 nm. In some embodiments, the average or mean diameters of the nanoparticles is no greater than about 150 nm. In some embodiments, the average or mean diameters of the nanoparticles is no greater than about 100 nm. In some embodiments, the average or mean diameter of the nanoparticles is about 20 to about 400 nm. In some embodiments, the average or mean diameter of the nanoparticles is about 40 to about 200 nm. In some embodiments, the nanoparticles are sterile-filterable. In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm.

In some embodiments, the composition comprises nanoparticles with diameter of no greater than about 1000 nanometers (nm), such as no greater than about any of 900, 800, 700, 600, 500, 400, 300, 200, and 100 nm. In some embodiments, the diameters of the nanoparticles in the composition are no greater than about 200 nm. In some embodiments, the diameters of the nanoparticles in the compositions are no greater than about 150 nm. In some embodiments, the diameters of the nanoparticles in the compositions are no greater than about 100 nm. In some embodiments, the nanoparticles in the composition described herein have a diameter of no greater than about 200 nm, including for example no greater than about any one of 190, 180, 170, 160, 150, 140, 130, 120, 110, 100, 90, 80, 70, or 60 nm.

In some embodiments, at least about 50% (for example at least about any one of 60%, 70%, 80%, 90%, 95%, or 99%) of all the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of all the nanoparticles in the composition fall within the range of about 20 to about 200 nm, including for example any one of about 30 to about 180 nm, and any one of about 40 to about 150, about 50 to about 120, and about 60 to about 100 nm.

In some embodiments, the carrier protein has sulfhydral groups that can form disulfide bonds. In some embodiments, at least about 5% (including for example at least about any one of 10%, 15%, 20%, 25%, 30%, 40%, 50%, 60%, 70%, 80%, or 90%) of the carrier protein in the nanoparticle portion of the composition are crosslinked (for example crosslinked through one or more disulfide bonds).

In some embodiments, the nanoparticles comprise the colchicine or thiocolchicine dimer (or taxane) coated with a carrier protein, such as albumin (e.g., human serum albumin). In some embodiments, the composition comprises colchicine or thiocolchicine dimer (or taxane) in both nanoparticle and non-nanoparticle forms, wherein at least about any one of 50%, 60%, 70%, 80%, 90%, 95%, or 99% of the colchicine or thiocolchicine dimer (or taxane) in the composition are in nanoparticle form. In some embodiments, the colchicine or thiocolchicine dimer (or taxane) in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of colchicine or thiocolchicine dimer (or taxane) that is substantially free of polymeric materials (such as polymeric matrix).

In some embodiments, the nanoparticle composition is substantially free (such as free) of surfactants (such as Cremophor®, Tween 80, or other organic solvents used for the administration of water insoluble drugs). In some embodiments, the nanoparticle composition contains less than about any one of 20%, 15%, 10%, 7.5%, 5%, 2.5%, or 1% organic solvent. In some embodiments, the weight ratio of carrier protein (such as albumin) and colchicine or thiocolchicine dimer (or taxane) in the nanoparticle composition is about 18:1 or less, such as about 15:1 or less, for example about 10:1 or less. In some embodiments, the weight ratio of carrier protein (such as albumin) and colchicine or thiocolchicine dimer (or taxane) in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of carrier protein and colchicine or thiocolchicine dimer (or taxane) in the nanoparticle portion of the composition is about any one of 1:2, 1:3, 1:4, 1:5, 1:10, 1:15, or less.

In some embodiments, the nanoparticle composition comprises one or more of the above characteristics.

The nanoparticles described herein may be present in a dry formulation (such as lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

The term "proteins" refers to polypeptides or polymers of amino acids of any length (including full length or fragments), which may be linear or branched, comprise modified amino acids, and/or be interrupted by non-amino acids. The term also encompasses an amino acid polymer that has been modified naturally or by intervention; for example, disulfide bond formation, glycosylation, lipidation, acetylation, phosphorylation, or any other manipulation or modification. Also included within this term are, for example, polypeptides containing one or more analogs of an amino acid (including, for example, unnatural amino acids, etc.), as well as other modifications known in the art. The proteins described herein may be naturally occurring, i.e., obtained or derived from a natural source (such as blood), or synthesized (such as chemically synthesized or by synthesized by recombinant DNA techniques).

Examples of suitable carrier proteins include proteins normally found in blood or plasma, which include, but are not limited to, albumin, immunoglobulin including IgA, lipoproteins, apolipoprotein B, alpha-acid glycoprotein, beta-2-macroglobulin, thyroglobulin, transferin, fibronectin, factor VII, factor VIII, factor IX, factor X, and the like. In some embodiments, the carrier protein is non-blood protein, such as casein, a-lactalbumin, and β-lactoglobulin. The carrier proteins may either be natural in origin or synthetically prepared. In some embodiments, the pharmaceutically acceptable carrier comprises albumin, such as human serum albumin. Human serum albumin (HSA) is a highly soluble globular protein of Mᵣ 65K and consists of 585 amino acids. HSA is the most abundant protein in the plasma and accounts for 70-80 % of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolumic shock (see, e.g., Tullis, JAMA, 237, 355-360, 460-463, (1977)) and Houser et al., Surgery, Gynecology and Obstetrics, 150, 811-816 (1980)) and in conjunction with exchange transfusion in the treatment of neonatal hyperbilirubinemia (see, e.g., Finlayson, Seminars in Thrombosis and Hemostasis, 6, 85-120, (1980)). Other albumins are contemplated, such as bovine serum albumin. Use of such non-human albumins could be appropriate, for example, in the context of use of these compositions in non-human mammals, such as the veterinary (including domestic pets and agricultural context).

Human serum albumin (HSA) has multiple hydrophobic binding sites (a total of eight for fatty acids, an endogenous ligand of HSA) and binds a diverse set of taxanes, especially neutral and negatively charged hydrophobic compounds. Two high affinity binding sites have been proposed in subdomains IIA and IIIA of HSA, which are highly elongated hydrophobic pockets with charged lysine and arginine residues near the surface which function as attachment points for polar ligand features (see, e.g., Fehske et al., Biochem. Pharmcol., 30, 687-92 (198a). In addition, docetaxel has been shown to bind to human plasma proteins (see, e.g., Urien et al., Invest. New Drugs,14(b), 147-51 (1996)).

The carrier protein (such as albumin) in the composition generally serves as a carrier for the colchicine or thiocolchicine dimer (or taxane), i.e., the carrier protein in the composition makes the colchicine or thiocolchicine dimer (or taxane) more readily suspendable in an aqueous medium or helps maintain the suspension as compared to compositions not comprising a carrier protein. This can avoid the use of toxic solvents (or surfactants) for solubilizing the colchicine or thiocolchicine dimer (or taxane), and thereby can reduce one or more side effects of administration of the colchicine or thiocolchicine dimer (or taxane) into an individual (such as a human). Thus, in some embodiments, the composition described herein is substantially free (such as free) of surfactants, such as Cremophor (including Cremophor EL® (BASF)). In some embodiments, the nanoparticle composition is substantially free (such as free) of surfactants. A composition is "substantially free of Cremophor" or "substantially free of surfactant" if the amount of Cremophor or surfactant in the composition is not sufficient to cause one or more side effect(s) in an individual when the nanoparticle composition is administered to the individual.

The amount of carrier protein in the composition described herein will vary depending on other components in the composition. In some embodiments, the composition comprises a carrier protein in an amount that is sufficient to stabilize the colchicine or thiocolchicine dimer (or taxane) in an aqueous suspension, for example, in the form of a stable colloidal suspension (such as a stable suspension of nanoparticles). In some embodiments, the carrier protein is in an amount that reduces the sedimentation rate of the colchicine or thiocolchicine dimer (or taxane) in an aqueous medium. For particle-containing compositions, the amount of the carrier protein also depends on the size and density of nanoparticles of the colchicine or thiocolchicine dimer (or taxane).

A colchicine or thiocolchicine dimer (or taxane) is "stabilized" in an aqueous suspension if it remains suspended in an aqueous medium (such as without visible precipitation or sedimentation) for an extended period of time, such as for at least about any of 0.1, 0.2, 0.25, 0.5, 1, 2, 3, 4, 5, 6, 7, 8, 9, 10, 11, 12, 24, 36, 48, 60, or 72 hours. The suspension is generally, but not necessarily, suitable for administration to an individual (such as human). Stability of the suspension is generally (but not necessarily) evaluated at a storage temperature (such as room temperature (such as 20-25 °C) or refrigerated conditions (such as 4°C)). For example, a suspension is stable at a storage temperature if it exhibits no flocculation or particle agglomeration visible to the naked eye or when viewed under the optical microscope at 1000 times, at about fifteen minutes after preparation of the suspension. Stability can also be evaluated under accelerated testing conditions, such as at a temperature that is higher than about 40 °C.

In some embodiments, the carrier protein is present in an amount that is sufficient to stabilize the colchicine or thiocolchicine dimer (or taxane) in an aqueous suspension at a certain concentration. For example, the concentration of the colchicine or thiocolchicine dimer (or taxane) in the composition is about 0.1 to about 100 mg/ml, including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg /ml. In some embodiments, the concentration of the colchicine or thiocolchicine dimer (or taxane) is at least about any of 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, and 50 mg/ml. In some embodiments, the carrier protein is present in an amount that avoids use of surfactants (such as Cremophor), so that the composition is free or substantially free of surfactant (such as Cremophor).

In some embodiments, the composition, in liquid form, comprises from about 0.1% to about 50% (w/v) (e.g. about 0.5% (w/v), about 5% (w/v), about 10% (w/v), about 15% (w/v), about 20% (w/v), about 30% (w/v), about 40% (w/v), or about 50% (w/v)) of carrier protein. In some embodiments, the composition, in liquid form, comprises about 0.5% to about 5% (w/v) of carrier protein.

In some embodiments, the weight ratio of carrier protein, e.g., albumin, to the colchicine or thiocolchicine dimer (or taxane) in the nanoparticle composition is such that a sufficient amount of the colchicine or thiocolchicine dimer (or taxane) binds to, or is transported by, the cell. While the weight ratio of carrier protein to the colchicine or thiocolchicine dimer (or taxane) will have to be optimized for different carrier protein and the colchicine or thiocolchicine dimer (or taxane) combinations, generally the weight ratio of carrier protein, e.g., albumin, to the colchicine or thiocolchicine dimer (or taxane) (w/w) is about 0.01:1 to about 100:1, about 0.02:1 to about 50:1, about 0.05:1 to about 20:1, about 0.1:1 to about 20:1, about 1:1 to about 18:1, about 2:1 to about 15:1, about 3:1 to about 12:1, about 4:1 to about 10:1, about 5:1 to about 9:1, or about 9:1. In some embodiments, the carrier protein to the colchicine or thiocolchicine dimer (or taxane) weight ratio is about any of 18:1 or less, 15:1 or less, 14:1 or less, 13:1 or less, 12:1 or less, 11:1 or less, 10:1 or less, 9:1 or less, 8:1 or less, 7:1 or less, 6:1 or less, 5:1 or less, 4:1 or less, and 3:1 or less.

In some embodiments, the carrier protein allows the composition to be administered to an individual (such as human) without significant side effects. In some embodiments, the carrier protein (such as albumin) is in an amount that is effective to reduce one or more side effects of administration of the colchicine or thiocolchicine dimer (or taxane) to a human. The term "reducing one or more side effects of administration of the colchicine or thiocolchicine dimer (or taxane)" refers to reduction, alleviation, elimination, or avoidance of one or more undesirable effects caused by the colchicine or thiocolchicine dimer (or taxane), as well as side effects caused by delivery vehicles (such as solvents that render the colchicine or thiocolchicine dimer (or taxane) suitable for injection) used to deliver the colchicine or thiocolchicine dimer (or taxane). Such side effects include, for example, myelosuppression, neurotoxicity, hypersensitivity, inflammation, venous irritation, phlebitis, pain, skin irritation, peripheral neuropathy, neutropenic fever, anaphylactic reaction, venous thrombosis, extravasation, and combinations thereof. These side effects, however, are merely exemplary and other side effects, or combination of side effects, associated with the colchicine or thiocolchicine dimer (or taxane) can be reduced.

In some embodiments, the colchicine or thiocolchicine dimer is IND-5404. In some embodiments, the colchicine or thiocolchicine dimer is comprised in Nab-5404.

In some embodiments, the taxane is comprised in Abraxane®. Abraxane® is a formulation of paclitaxel stabilized by human albumin USP, which can be dispersed in directly injectable physiological solution. When dispersed in a suitable aqueous medium such as 0.9% sodium chloride injection or 5% dextrose injection, Abraxane® forms a stable colloidal suspension of paclitaxel. The mean particle size of the nanoparticles in the colloidal suspension is about 130 nanometers. Since HSA is freely soluble in water, Abraxane® can be reconstituted in a wide range of concentrations ranging from dilute (0.1 mg/ml paclitaxel) to concentrated (20 mg/ml paclitaxel), including for example about 2 mg/ml to about 8 mg/ml, about 5 mg/ml.

Methods of making nanoparticle compositions are known in the art. For example, nanoparticles containing taxanes (such as paclitaxel) and carrier protein (such as albumin) can be prepared under conditions of high shear forces (e.g., sonication, high pressure homogenization, or the like). These methods are disclosed in, for example, U.S. Pat. Nos. 5,916,596; 6,506,405; 6,749,868, and 6,537,579 and also in U.S. Pat. Pub. No. 2005/0004002, 2007/0082838, 2006/0263434and PCT Application WO08/137148.

Briefly, the colchicine or thiocolchicine dimer (or taxane) is dissolved in an organic solvent, and the solution can be added to a human serum albumin solution. The mixture is subjected to high pressure homogenization. The organic solvent can then be removed by evaporation. The dispersion obtained can be further lyophilized. Suitable organic solvent include, for example, ketones, esters, ethers, chlorinated solvents, and other solvents known in the art. For example, the organic solvent can be methylene chloride or chloroform/ethanol (for example with a ratio of 1:9, 1:8, 1:7, 1:6, 1:5, 1:4, 1:3, 1:2, 1:1, 2:1, 3:1, 4:1, 5:1, 6:1, 7:1, 8:1, or 9:1.

### Other components in the nanoparticle compositions

The nanoparticles described herein can be present in a composition that includes other agents, excipients, or stabilizers. For example, to increase stability by increasing the negative zeta potential of nanoparticles, certain negatively charged components may be added. Such negatively charged components include, but are not limited to bile salts of bile acids consisting of glycocholic acid, cholic acid, chenodeoxycholic acid, taurocholic acid, glycochenodeoxycholic acid, taurochenodeoxycholic acid, litocholic acid, ursodeoxycholic acid, dehydrocholic acid and others; phospholipids including lecithin (egg yolk) based phospholipids which include the following phosphatidylcholines: palmitoyloleoylphosphatidylcholine, palmitoyllinoleoylphosphatidylcholine, stearoyllinoleoylphosphatidylcholine stearoyloleoylphosphatidylcholine, stearoylarachidoylphosphatidylcholine, and dipalmitoylphosphatidylcholine. Other phospholipids including L-a-dimyristoylphosphatidylcholine (DMPC), dioleoylphosphatidylcholine (DOPC), distearyolphosphatidylcholine (DSPC), hydrogenated soy phosphatidylcholine (HSPC), and other related compounds. Negatively charged surfactants or emulsifiers are also suitable as additives, e.g., sodium cholesteryl sulfate and the like.

In some embodiments, the composition is suitable for administration to a human. In some embodiments, the composition is suitable for administration to a mammal such as, in the veterinary context, domestic pets and agricultural animals. There are a wide variety of suitable formulations of the nanoparticle composition (see, e.g., U.S. Pat. Nos. 5,916,596 and 6,096,331). The following formulations and methods are merely exemplary and are in no way limiting. Formulations suitable for oral administration can consist of (a) liquid solutions, such as an effective amount of the compound dissolved in diluents, such as water, saline, or orange juice, (b) capsules, sachets or tablets, each containing a predetermined amount of the active ingredient, as solids or granules, (c) suspensions in an appropriate liquid, and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Examples of suitable carriers, excipients, and diluents include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline solution, syrup, methylcellulose, methyl- and propylhydroxybenzoates, talc, magnesium stearate, and mineral oil. The formulations can additionally include lubricating agents, wetting agents, emulsifying and suspending agents, preserving agents, sweetening agents or flavoring agents.

Formulations suitable for parenteral administration include aqueous and non-aqueous, isotonic sterile injection solutions, which can contain anti-oxidants, buffers, bacteriostats, and solutes that render the formulation compatible with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipients, for example, water, for injections, immediately prior to use. Extemporaneous injection solutions and suspensions can be prepared from sterile powders, granules, and tablets of the kind previously described. Injectable formulations are preferred.

In some embodiments, the composition is formulated to have a pH range of about 4.5 to about 9.0, including for example pH ranges of any of about 5.0 to about 8.0, about 6.5 to about 7.5, and about 6.5 to about 7.0. In some embodiments, the pH of the composition is formulated to no less than about 6, including for example no less than about any of 6.5, 7, or 8 (such as about 8). The composition can also be made to be isotonic with blood by the addition of a suitable tonicity modifier, such as glycerol.

### Kits

The invention also provides kits for use in the instant methods. Kits of the invention include one or more containers comprising drugs described herein, and in some embodiments, further comprise instructions for use in accordance with any of the methods described herein. The kit may further comprise a description of selection an individual suitable or treatment. Instructions supplied in the kits of the invention are typically written instructions on a label or package insert (e.g., a paper sheet included in the kit), but machine-readable instructions (e.g., instructions carried on a magnetic or optical storage disk) are also acceptable.

In some embodiments, the kit comprises a) a colchicine or thiocolchicine dimer, b) an anti-VEGF antibody, and c) instructions for administering the nanoparticles and the other agents simultaneously and/or sequentially, for treatment of a proliferative disease (such as cancer). In some embodiments, the kit further comprises a taxane. taxane is any of paclitaxel, docetaxel, and ortataxel. In some embodiments, the colchicine or thiocolchicine dimer (and/or taxane) are in the form of nanoparticles, such as nanoparticles described herein.

It is understood that the kit may comprise one distinct composition or two or more compositions wherein one composition comprises drugs described herein.

The kits of the invention are in suitable packaging. Suitable packaging include, but is not limited to, vials, bottles, jars, flexible packaging (e.g., seled Mylar or plastic bags), and the like. Kits may optionally provide additional components such as buffers and interpretative information.

The instructions generally include information as to dosage, dosing schedule, and route of administration for the intended treatment. The containers may be unit doses, bulk packages (e.g., multi-dose packages) or sub-unit doses. For example, kits may be provided that contain sufficient dosages of the taxane (such as taxane) as disclosed herein to provide effective treatment of an individual for an extended period, such as any of a week, 2 weeks, 3 weeks, 4 weeks, 6 weeks, 8 weeks, 3 months, 4 months, 5 months, 7 months, 8 months, 9 months, or more. Kits may also include multiple unit doses of the drugs and pharmaceutical compositions and instructions for use and packaged in quantities sufficient for storage and use in pharmacies, for example, hospital pharmacies and compounding pharmacies.

The invention will now be described in greater detail by reference to the following examples. The following examples further illustrate the invention.

### EXAMPLES

### Example 1. Sequence-dependent enhancement of antitumor activity of the vascular disrupting agent ABI-011 by nab-paclitaxel and bevacizumab

Background: The tumor vasculature is an established target for anticancer therapies. Vascular disrupting agents (VDAs) compromise established tumor vasculature and have the potential to destroy tumor masses as well as preventing progression. ABI-011, a nanoparticle composition comprising a thiocolchicine dimer (IND5404) and human serum albumin, is a potent VDA with antitubulin and topoisomerase 1 inhibitor properties. ABI-011 displayed significant anti-tumor activity in mouse xenograft models of human breast, colon, prostate, and ovarian carcinoma. In this study, the importance of dose, schedule, and sequence for the combination of ABI-011 and nab-paclitaxel (Abraxane®) or bevacizumab (Avastin) was evaluated in mice bearing xenografted human tumors.

Methods: Subcutaneous human breast (MDA-MB-231), colon (HT29) and prostate (PC3) tumors were grown in athymic nude mice and treated intravenously (IV) with sub-optimal dose of ABI-011 alone at 20 mg/kg (q4dx3, q4dx2, or qdx1) and in combination with nab-paclitaxel (5 mg/kg, q4dx3, IV) or bevacizumab (8 mg/kg, 2xwkly, IP). The effect of dose, schedule, and sequence of combination regimen on therapeutic efficacy was tested. ABI-011 was administered 24 h before, concurrent with, or 24 h after nab-paclitaxel or bevacizumab treatment.

### Results:

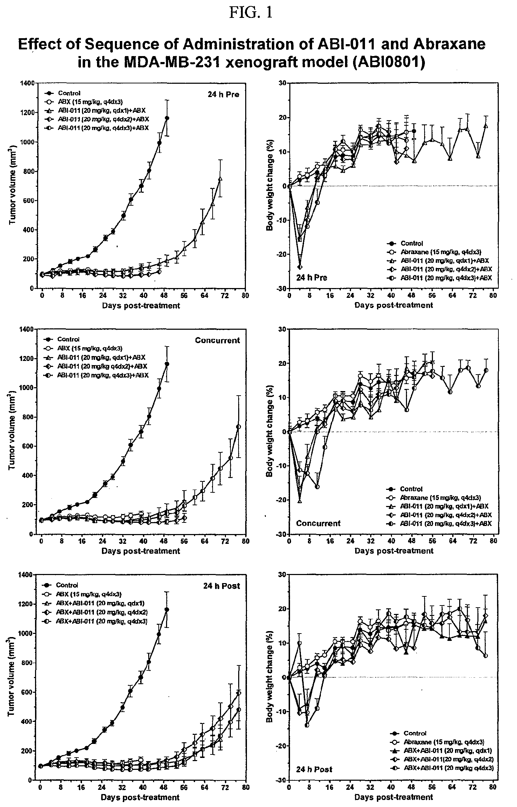

Figure 1 (as reference) shows the effect of sequence of administration of ABI-011 and Abraxane® in the MDA-MB-231 xenograft model.

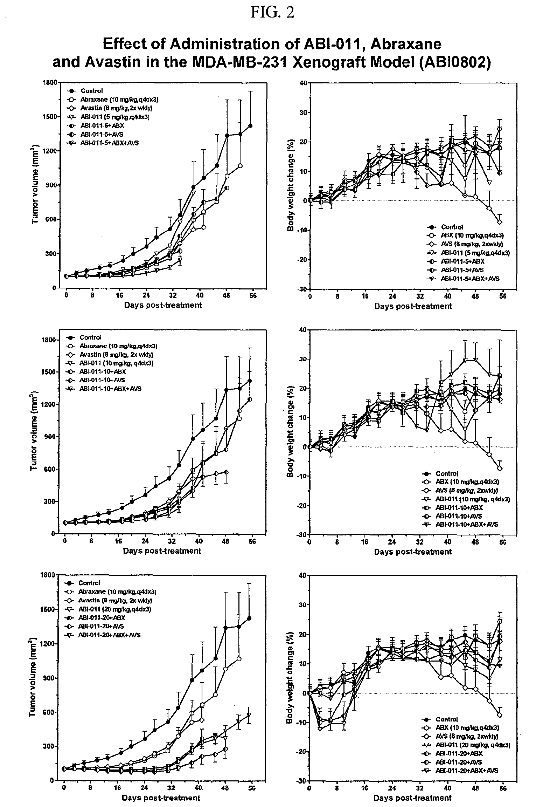

Figure 2 shows the effect of administration of ABI-011, Abraxane®, and Avastin in the MDA-MB-231 xenograft model.

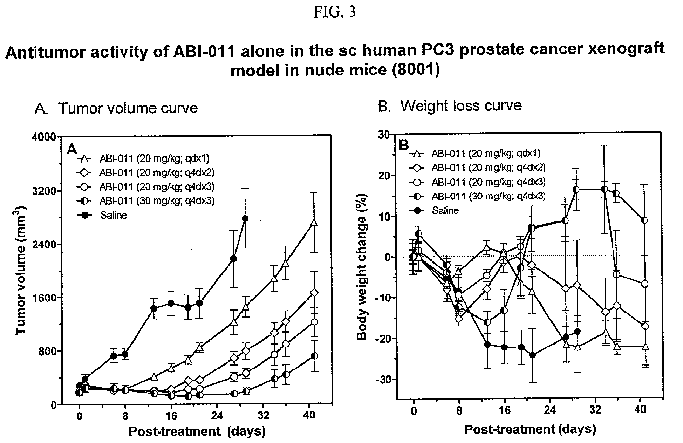

Figure 3 (as reference) shows the anti-tumor activity of ABI-011 alone in the s.c. human PC3 prostate cancer xenograft model in nude mice.

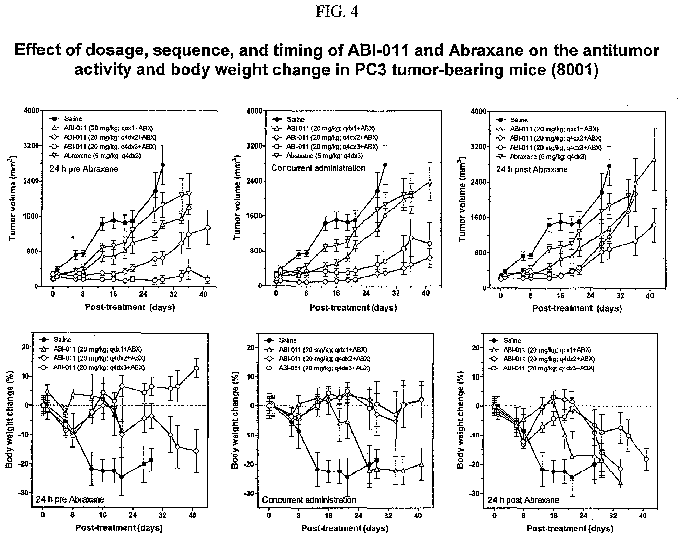

Figure 4 (as reference) shows the effect of dosage, sequence, and timing of ABI-011 and Abraxane® on the antitumor activity and body weight change in PC3 tumor-bearing mice.

Table 1 (as reference) summarizes the antitumor activity and body weight change in PC3 tumor-bearing mice treated with ABI-011 and ABI-011 plus Abraxane®. As shown in Table 1, Administration of ABI-011 alone and in combination with Abraxane® effectively delayed PC3 human tumor growth as well as caused dose-dependent reduction in weight loss observed in PC3 tumor-bearing mice. ABI-011 (20 mg/kg, q4dx2 or q4dx3 schedules) significantly enhanced the antitumor effect of Abraxane® (5 mg/kg, q4dx3) when administered 24 h prior to or concurrently with Abraxane® (p <0.001 versus Abraxane®).

**Table 1. (as reference)**

| **Agent** | **Dose (mg/kg)** | **Schedule** | **TGI (%)^{a}** | **BWC (%)^{b}** |
|---|---|---|---|---|
| Saline | 0 | q4dx3 | - | -24.4 |
| Abraxane (ABX) | 5 | q4dx3 | 35.3 | |
| ABI-011 | 20 | qdx1 | 50.7 | -8.6 |
| ABI-011 | 20 | q4dx2 | 77.3 | -2.1 |
| ABI-011 | 20 | q4dx3 | 90.2 | +6.6 |
| ABI-011 | 30 | q4dx3 | 99.6 | +6.6 |
| *24 h pre* | | | | |
| ABI-011 + ABX | 20+5 | qdx1 + q4dx3 | 52.3 | -8.4 |
| ABI-011 + ABX | 20+5 | q4dx2 + q4dx3 | 85.5 | -9.8 |
| ABI-011 + ABX | 20+5 | q4dx3 + q4dx3 | 100 | +6.6 |
| *Concurrent* | | | | |
| ABI-011 + ABX | 20+5 | qxd1 + q4dx3 | 43.5 | -5.1 |
| ABI-011 + ABX | 20+5 | q4dx2 + q4dx3 | 92.5 | +3.7 |
| ABI-011 + ABX | 20+5 | q4dx3 + q4dx3 | 87.7 | +5.1 |
| *24 h post* | | | | |
| ABI-011 + ABX | 20+5 | qxd1 + q4dx3 | 56.8 | -17.1 |
| ABI-011 + ABX | 20+5 | q4dx2 + q4dx3 | 62.8 | +2.2 |
| ABI-011 + ABX | 20+5 | q4dx3 + q4dx3 | 75.4 | -3.5 |

| | | | | |
|---|---|---|---|---|
| ^{a}TGI, tumor growth inhibition on day 29; ^{b}BWC, percent body weight change on day 21. | | | | |

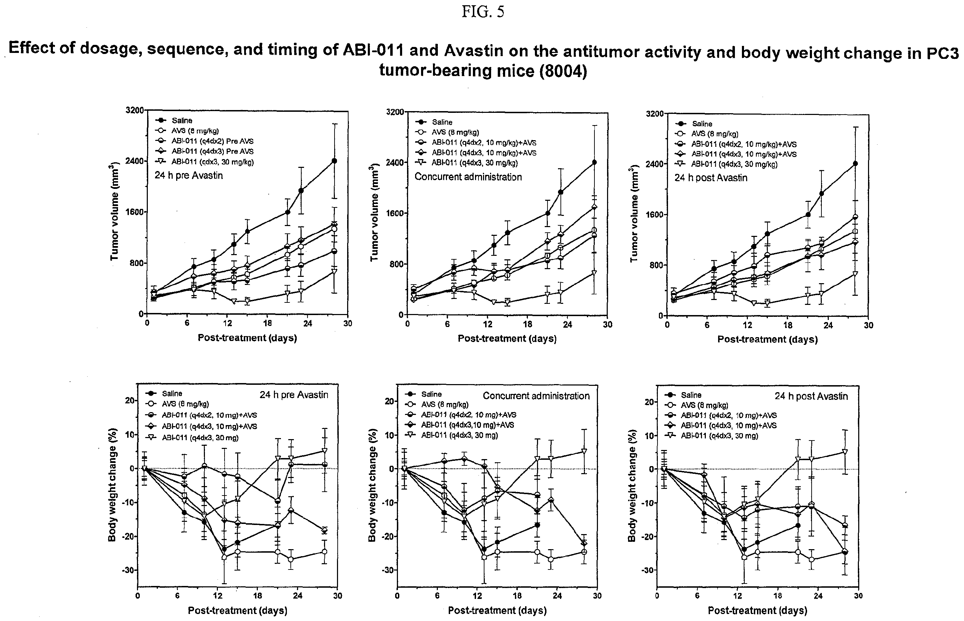

Figure 5 (as reference) shows the effect of dosage, sequence, and timing of ABI-011 and Avastin on the antitumor activity and body weight change in PC3.

Table (as reference) summarizes effect of dosage, sequence, and timing of ABI-011 and Avastin on the antitumor activity and body weight change in PC3 tumor-bearing mice. As shown in Table 2, Treatment with ABI-011 and Avastin resulted in reduction in anti-tumor activity and protection from body weight loss induced by PC3 cells.

**Table 2. (as reference)**

| Agent | Dose (mg/kg) | Schedule | TGI (%)^{a} | BWC (%)^{b} |
|---|---|---|---|---|
| Saline | 0 | q4dx3 | - | -16.6 |
| Avastin | 8 | 2x/wk | 52.9 | -24.6 |
| ABI-011 | 10 | q4dx3 | 37.9 | -13.9 |
| ABI-011 | 30 | q4dx3 | 95.9 | +2.98 |
| *24 h pre* | | | | |
| ABI-011 + AVS | 10+8 | q4dx2 + 2x/wk | 70.8 | -9.5 |
| ABI-011 + AVS | 10+8 | q4dx3 + 2x/wk | 49.8 | -16.9 |
| *Concurrent* | | | | |
| ABI-011 + AVS | 10+8 | q4dx2 + 2x/wk | 69.8 | -7.6 |
| ABI-011 + AVS | 10+8 | q4dx3 + 2x/wk | 40.3 | -12.2 |
| *24 h post* | | | | |
| AVS + ABI-011 | 8+10 | 2x/wk + q4dx2 | 51.6 | -11.0 |
| AVS + ABI-011 | 8+10 | 2x/wk + q4dx3 | 58.6 | -13.3 |

| | | | | |
|---|---|---|---|---|
| ^{a}TGI, tumor growth inhibition on day 23; ^{b}BWC, percent body weight change on day 21. | | | | |

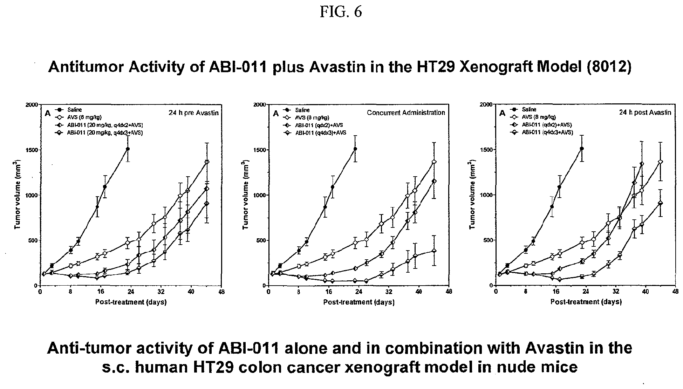

Figure 6 (as reference) shows the antitumor activity of ABI-011 plus Avastin in the HT29 xenograft model. The different groups tested are provided in Table 3.

**Table 3. (as reference)**

| **Dosing Groups (8012)** | |
|---|---|
| Groups | Treatment |
| **A** | 0.9% saline q4dx3 |
| **B** | ABI-011 20 mg/kg q4dx3 |
| **C** | Avastin 0.2 mg/mouse 2x/week |
| **D** | ABI-011 20 mg/kg q4dx3+ Avastin 0.2 mg/mouse 24hrs after |
| **E** | ABI-011 20 mg/kg q4dx3+ Avastin 0.2 mg/mouse concurrent |
| **F** | ABI-011 20 mg/kg q4dx3+ Avastin 0.2 mg/mouse 24hrs prior |
| **G** | ABI-011 20 mg/kg q4dx2 |
| **H** | ABI-011 20 mg/kg q4dx2+ Avastin 0.2 mg/mouse 24hrs after |
| **I** | ABI-011 20 mg/kg q4dx2+ Avastin 0.2 mg/mouse concurrent |
| **J** | ABI-011 20 mg/kg q4dx2+ Avastin 0.2 mg/mouse 24hrs prior |
| **K** | ABI-011 20 mg/kg q4dx1 |

Table 4 (as reference) summarizes anti-tumor activity of ABI-011 alone and in combination with Avastin in the s.c. human HT29 colon cancer xenograft model in nude mice.

**Table 4. (as reference)**

| Agent | Dose (mg/kg) | Schedule | TGI (%)^{a} | Three-TDT (days) | BWC (%)^{b} |
|---|---|---|---|---|---|
| Saline | 0 | q4dx3 | - | 8.2 | |
| Avastin | 8 | 2x/wk | 75.3 | 19.3 | |
| ABI-011 | 20 | q4dx1 | -23.2 | 10.7 | |
| ABI-011 | 20 | q4dx2 | 31.2 | 16.7 | |
| ABI-011 | 20 | q4dx3 | 91.3 | 26.2 | |
| *24 h pre* | | | | | |
| ABI-011 + AVS | 20+8 | q4dx2 + 2x/wk | 92.7 | 30.1 | |
| ABI-011 + AVS | 20+8 | q4dx3 + 2x/wk | 99.2 | 34.0 | |
| *Concurrent* | | | | | |
| ABI-011 + AVS | 20+8 | q4dx2 + 2x/wk | 96.0 | 31.2 | |
| ABI-011 + AVS | 20+8 | q4dx3 + 2x/wk | 100 | >45 | |
| *24 h post* | | | | | |
| AVS + ABI-011 | 8+20 | 2x/wk + q4dx2 | 90.2 | 27.4 | |
| AVS + ABI-011 | 8+20 | 2x/wk + q4dx3 | 100 | 33.7 | |

| | | | | | |
|---|---|---|---|---|---|
| ^{a}TGI, tumor growth inhibition on day 23; ^{b}BWC, percent body weight change on day 23. | | | | | |

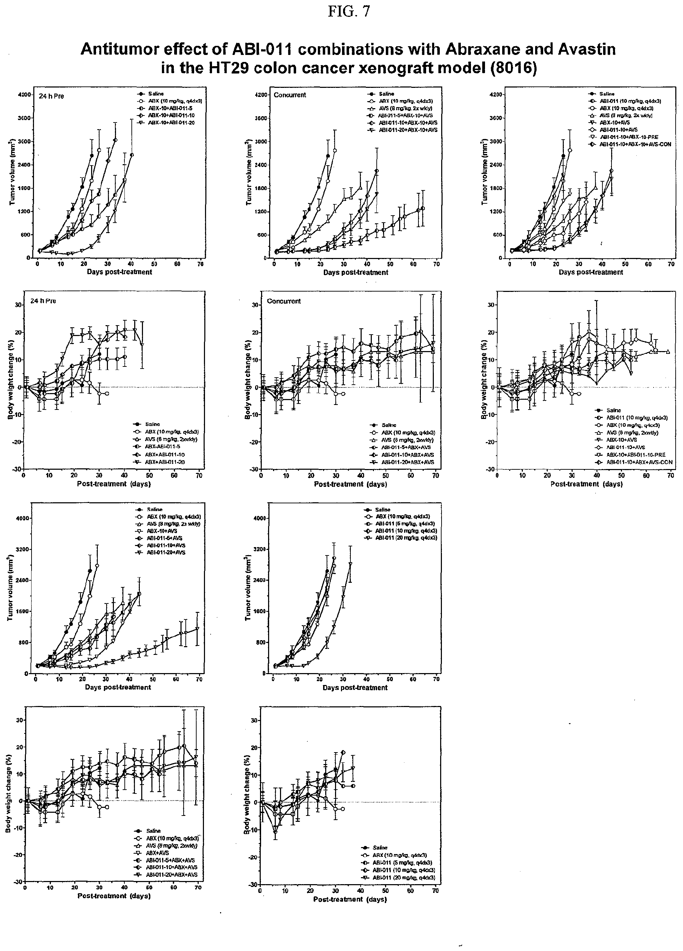

Figure 7 shows the antitumor effect of ABI-011 combinations with Abraxane® and Avastin in the HT29 colon cancer xenograft model. The different groups tested are provided in Table 5.

**Table 5.**

| | **8016 TREATMENT GROUPS (N = 5)** |
|---|---|
| **A** | Saline |
| **B** | Abraxane 10 mg/kg (q4dx3) |
| **C** | Avastin 8 mg/kg (2x/wk) |
| **D** | Abraxane 10 mg/kg (q4dx3) + Avastin 8 mg/kg (2x/wk) |
| **E** | Nab-5404 20 mg/kg (q4dx3) |
| F | Nab-5404 10 mg/kg (q4dx3) |
| **G** | Nab-5404 5 mg/kg (q4dx3) |
| **H** | Nab-5404 20 mg/kg (q4dx3) + Abraxane 10 mg/kg (q4dx3) + Avastin 8 mg/kg (2x/wk) |
| **I** | Nab-5404 10 mg/kg (q4dx3) + Abraxane 10 mg/kg (q4dx3) + Avastin 8 mg/kg (2x/wk) |
| **J** | Nab-5404 5 mg/kg (q4dx3) + Abraxane 10 mg/kg (q4dx3) + Avastin 8 mg/kg (2x/wk) |
| **K** | Nab-5404 20 mg/kg (q4dx3) + Avastin 8 mg/kg (2x/wk) |
| **L** | Nab-5404 10mg/kg (q4dx3) + Avastin 8 mg/kg (2x/wk) |
| **M** | Nab-5404 5 mg/kg (q4dx3) + Avastin 8 mg/kg (2x/wk) |
| **N** | Abraxane 10 mg/kg (q4dx3) + Nab-5404 20 mg/kg 24 hours prior |
| **O** | Abraxane 10 mg/kg (q4dx3) + Nab-5404 10 mg/kg 24 hours prior |
| **P** | Abraxane 10 mg/kg (q4dx3) + Nab-5404 5 mg/kg 24 hours prior |

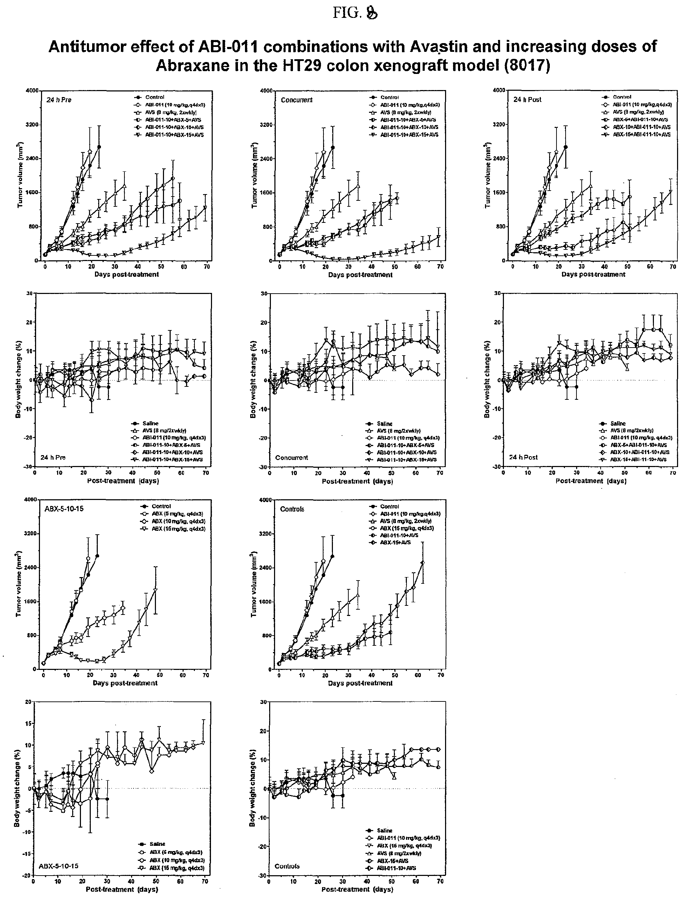

Figure 8 shows the antitumor effect of ABI-011 combination with Avastin and increasing doses of Abraxane® in the HT29 colon cancer xenograft model. The different groups tested are provided in Table 6.

**Table 6.**

| **DOSING GROUPS (8017)** | | |
|---|---|---|
| **A** | Saline | |
| **B** | Nab-5404 (10 mg/kg, q4dx3) | |
| **C** | ABX (5 mg/kg, q4dx3) | |
| **D** | ABX (10 mg/kg, q4dx3) | |
| **E** | ABX (15 mg/kg, q4dx3) | |
| **F** | AVS (0.2 mg/kg, twice weekly) | |
| | | |
| **G** | Nab-5404 **10** + ABX-**5**, + AVS | Nab-5404 administered 24 hours prior to ABX |
| **H** | Nab-5404 **10** + ABX-**10**, + AVS | Nab-5404 administered 24 hours prior to ABX |
| **I** | Nab-5404 **10** + ABX-**15**, + AVS | Nab-5404 administered 24 hours prior to ABX |
| **J** | Nab-5404 **10** + ABX-**5**, + AVS | ABX and Nab-5404 administered concurrently |
| **K** | Nab-5404 **10** + ABX-**10**, + AVS | ABX and Nab-5404 administered concurrently |
| **L** | Nab-5404 **10 +** ABX-**15**, + AVS | ABX and Nab-5404 administered concurrently |
| **M** | ABX-**5** + Nab-5404-**10** + AVS | ABX administered 24 hours prior to Nab-5404 |
| **N** | ABX-**10**+ Nab-5404-**10** + AVS | ABX administered 24 hours prior to Nab-5404 |
| **O** | ABX-**15**+ Nab-5404-**10** + AVS | ABX administered 24 hours prior to Nab-5404 |
| **P** | ABX-**15** + AVS | |
| **Q** | Nab-5404-**10** + AVS | |
| | All IV dosing: q4dx3 | |
| | All Avastin (AVS) dousing: IP twice weekly at 0.2 mg/mouse | |

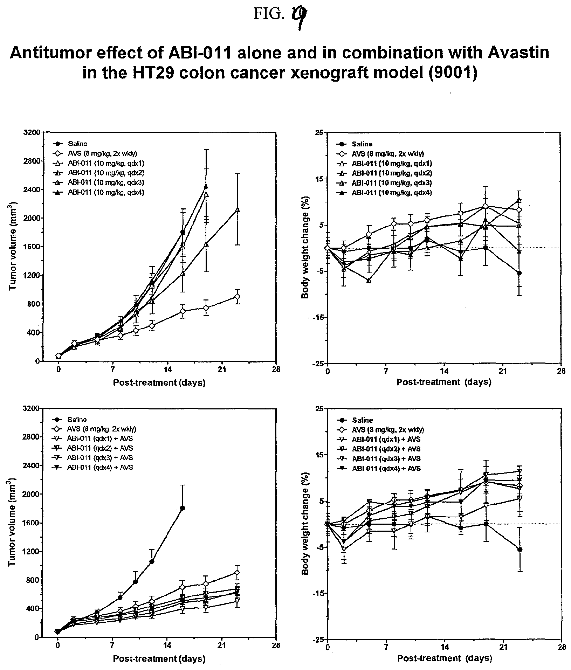

Figure 9 (as reference) shows antitumor effect of ABI-011 alone and in combination with Avastin in the HT29 colon cancer xenograft model. The different test groups are provided in Table 7.

**Table 7. (as reference)**

| **DOSING GROUPS (9001)** | |
|---|---|
| **A** | Saline |
| **B** | ABI-011 qdx1 |
| **C** | ABI-011 qdx2 |
| **D** | ABI-011 qdx3 |
| **E** | ABI-011 qdx4 |
| **F** | Avastin |
| **G** | ABI-011 qdx1 + Avastin |
| **H** | ABI-011 qdx2 + Avastin |
| **I** | ABI-011 qdx3 + Avastin |
| **J** | ABI-011 qdx4 + Avastin |
| | |
| | ABI-011 dosed IV at 10 mg/kg |
| | Avastin dosed IP twice weekly at 0.2 mg/mouse |

In summary, significant dose-dependent tumor growth inhibition (TGI) was observed in all xenograft models treated with ABI-011. The order of sensitivity of these three human xenografts to ABI-011 was MDA-MB-231 > PC3 > HT29. For MDA-MB-231, q4dx3, q4dx2, and qdx1 resulted in 80%, 78%, and 76% TGI, respectively. For PC3, q4dx3, q4dx2, and qdx1 resulted in 90%, 77%, and 51% TGI, respectively. For HT29, q4dx3, q4dx2, and qdx1 resulted in 84%, 22%, and 0% TGI, respectively. When ABI-011 (20 mg/kg, q4dx3) was administered 24 h before, concurrently or 24 h after nab-paclitaxel (5 mg/kg, q4dx3) in the PC3 model, the combination resulted in TGI of 100%, 88%, and 75% respectively. When ABI-011 (20 mg/kg, q4dx3) was administered 24 h before, concurrently, or 24 h after bevacizumab (8 mg/kg, 2xwkly) in the HT29 model, the combination resulted in TGI of 90%, 96%, and 93%, respectively.

Discussion: ABI-011 alone and in combination with nab-paclitaxel demonstrated significant TGI in xenograft models. The optimal therapeutic efficacy was achieved when ABI-011 was administered 24 h before nab-paclitaxel or concurrent with bevacizumab. The combination data suggest that effective combination of Avastin, Abraxane®, and ABI-011 is feasible. The double or triple drug combination therapy would be expected to be more effective than monotherapy.

### Example 2. Pharmacokinetic and cardiovascular safety profile of ABI-011 in cynomolgus monkeys (as reference)

Background: ABI-011 is a thiocolchicine dimer with potent vascular disrupting and antitumor activities. Vascular disrupting agents (VDAs) have been shown to have side effects related to their cytotoxicity and tubulin-binding abilities at higher doses than are required for the tumor vascular shutdown. Consequently, adverse cardiac effects have been previously reported for tubulin-binding VDAs such as ZD6126. In this study, ABI-011 was examined for its pharmacokinetic (PK) properties and cardiopulmonary safety profile in cynomolgus monkeys.

Methods: In the multiple dose blood pharmacokinetic study, ABI-011 was intravenously administered over 30 min at 1.67, 2.5 and 3.33 mg/kg weekly for 3 weeks in male and female cynomolgus monkeys (n = 3-5 animals/sex/group). Blood samples were collected pre-dose, during, and after infusion on Study Days 1 and 15. Whole blood samples were analyzed for ABI-011 concentration using liquid-liquid extraction and a liquid chromatography with tandem mass spectrometry. In the cardiopulmonary safety pharmacology study, 5 male conscious telemetered cynomolgus monkeys were administered ABI-011 at 0, 1.67, 2.5 and 3.33 mg/kg weekly for 4 weeks following a 4×4 Latin Square dosage regimen. Parameters recorded by telemetry included arterial blood pressure, heart rate, respiratory rate, body temperature, electrocardiogram (ECG, lead II), and motor activity. Blood cardiac Troponin I and creatine kinase (CK) isoenzyme levels were also evaluated. In addition, ABI-011 was evaluated in an in vitro human ether-a-go-go (HERG) potassium channel patch-clamp assay using hERG-transfected HEK293 cells.

Results: In cynomolgus monkeys, ABI-011 exhibited dose proportional pharmacokinetics with respect to dose, large volume of distribution (Vz), rapid clearance with HL ranging from 0.36 to 2.9 h, and no accumulation following multiple dosing. In the cardiopulmonary safety study, all pulmonary and ECG evaluations were normal with no indication of conduction abnormalities or cardiac muscle toxicity. A dose-dependent transient hypertension beginning at approximately 1 h post dose and peaking around 4 h post dose was apparent. The levels of cardiac Troponin I and cardiac-specific CK-MB levels remained within normal limits. Furthermore, ABI-011 inhibited whole-cell hERG current at the clinically non-relevant dose (IC50 value of 31.8 µM).

Conclusion: ABI-011 displayed efficient tissue extraction from the central blood compartment. ABI-011 infusion once weekly for 3 wks was well-tolerated at all dose levels (1.67, 2.5, and 3.33 mg/kg) with a no-observed-adverse-effect level (NOAEL) determined at 1.67 mg/kg. In vitro and in vivo studies of ABI-011 show no evidence for an increased cardiovascular safety risk.

### Example 3. Vascular disrupting activity of ABI-011 (as reference)

Background: The tumor vasculature is an established target for anticancer therapies. ABI-011 is a thiocolchicine dimer with antitubulin and topisomerase 1 inhibitor properties. In this study, ABI-011 was examined for antiangiogenic as well as vascular disrupting activities (VDA).

Methods: The antiangiogenic and VDA effects of ABI-011 were examined in vitro using tubule formation assay with human umbilical vein endothelial cells (HUVEC) and in vivo using the standard chick embryonic chorioallantoic membrane (CAM) assay with 3-day old embryos (n = 18). The shell-less quail embryonic CAM assay was used to determine the time course of VDAs (ABI-011 vs. combretastatin A4 Phosphate [CA4P]). Quail embryos (n = 36) were exposed to ABI-011 (1 to 16 µg/ml) on day 7 and digital CAM images were acquired over a 60 min period for scoring VDA. Subcutaneous human colon (HT29) tumors were grown in athymic nude mice and treated intravenously (IV) with ABI-011 (20-40 mg/kg; q4dx3, IV) or CA4P (200 mg/kg; qdx4, IV). The animal weights and tumor measurements were recorded three times weekly and adverse observations recorded.

Results: In the tubule formation assay, ABI-011 significantly inhibited new microvessel formation and disrupted established microvessels even at the dose of 0.01 µg/ml. In the chicken embryonic CAM assay, ABI-011 demonstrated potent antiangiogenic activity in a concentration-dependent manner, with over 90% inhibition at 5 µg without affecting viability. In the quail embryonic CAM assay, ABI-011 exerted potent VDA in a concentration- and time-dependent manner. ABI-011 at concentration as low as 2 µg/CAM caused visible disruption of CAM vasculature at 60 min post-treatment, with the IC50 calculated to be 1.8 µg/ml. Higher concentrations caused increasing embryonic mortalities with an LD50 of 3.4 µg/ml. In contrast, the vascular disrupting agent CA4P demonstrated only modest VDA at concentrations much higher than the effective dose of ABI-011, with the IC50 of CA4P determined to be 13.1 µg/ml- close to its LD50 of 12.8 µg/ml. Against HT29 tumor model, ABI-011 was highly effective with greater than 80% tumor growth inhibition versus CA4P being ineffective.

Conclusion: ABI-011 exhibited potent and specific antiangiogenic and vascular disrupting activities both in vitro and in vivo. ABI-011 was determined to be a more potent vascular disrupting antitumor agent with better therapeutic index than CA4P.

Preferred embodiments of this invention are described herein, including the best mode known to the inventors for carrying out the invention. Embodiments of those preferred embodiments may become apparent to those of ordinary skill in the art upon reading the foregoing description. The inventors expect skilled artisans to employ such embodiments as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible embodiments thereof is encompassed by the invention unless otherwise indicated herein or otherwise clearly contradicted by context.

## Claims

1. A composition comprising a colchicine or thiocolchicine dimer for use in a method of treating a proliferative disease in an individual, wherein the method comprises further administration of an anti-VEGF antibody and a taxane.

2. The composition for use according to claim 1, wherein the colchicine or thiocochicine dimer is IDN-5404.

3. The composition for use according to claim 1 or 2, wherein the colchicine or thiocolchicine dimer is in the form of nanoparticles.

4. The composition for use according to claim 3, wherein the nanoparticles comprise the colchicine or thiocolchicine dimer and an albumin, preferably colchicine or thiocolchicine dimer coated with the albumin.

5. The composition for use according to claim 4, wherein the average diameter of the nanoparticles in the composition is no greater than 200 nm.

6. The composition for use according to any one of claims 1-5, wherein the taxane is in the form of nanoparticles.

7. The composition for use according to claim 6, wherein the taxane nanoparticle comprises taxane and an albumin, preferably taxane coated with the albumin.

8. The composition for use according to claim 6 or 7, wherein the average diameter of the taxane nanoparticles is no greater than 200 nm.

9. The composition for use according to claim 8, wherein the taxane is paclitaxel.

10. The composition for use according to any one of claims 1-9, wherein the colchicine or thiocolchicine dimer, the anti-VEGF antibody and the taxane are administered concurrently or simultaneously.

11. The composition for use according to any one of claims 1-10, wherein the method comprises intravenously administering at least one of the colchicine or thiocolchicine dimer, the anti-VEGF antibody, and the taxane.

12. The composition for use according to any one of claims 1-11, wherein the anti-VEGF antibody is bevacizumab.

13. The composition for use according to any one of claims 1-12, wherein the proliferative disease is cancer, preferably a vascular tumor, or any of breast cancer, colon cancer, or prostate cancer, or a solid tumor.

14. The composition for use according to any one of claims 1-13, wherein the individual is a human.

## Patentansprüche

1. Zusammensetzung, welche ein Colchicin- oder Thiocolchicindimer umfasst, zur Verwendung in einem Verfahren zum Behandeln einer proliferativen Erkrankung eines Individuums, wobei das Verfahren des Weiteren die Verabreichung eines Anti-VEGF-Antikörpers und eines Taxans umfasst.

2. Zusammensetzung zur Verwendung gemäß Anspruch 1, wobei das Colchicin- oder Thiocolchicindimer IDN-5404 ist.

3. Zusammensetzung zur Verwendung gemäß Anspruch 1 oder 2, wobei das Colchicin- oder Thiocolchicindimer in Form von Nanopartikeln vorliegt.

4. Zusammensetzung zur Verwendung gemäß Anspruch 3, wobei die Nanopartikeln das Colchicin oder Thiocolchicindimer und ein Albumin umfassen, vorzugsweise Colchicin- oder Thiocolchicindimer, welche mit dem Albumin überzogen sind.

5. Zusammensetzung zur Verwendung gemäß Anspruch 4, wobei der mittlere Durchmesser der Nanopartikel in der Zusammensetzung nicht größer als 200 nm ist.

6. Zusammensetzung zur Verwendung gemäß einem der Ansprüche 1-5, wobei das Taxan in Form von Nanopartikeln vorliegt.

7. Zusammensetzung zur Verwendung gemäß Anspruch 6, wobei das Taxan-Nanopartikel Taxan und ein Albumin umfasst, vorzugsweise Taxan, welches mit dem Albumin überzogen ist.

8. Zusammensetzung zur Verwendung gemäß Anspruch 6 oder 7, wobei der durchschnittliche Durchmesser der Taxan-Nanopartikel nicht größer als 200 nm ist.

9. Zusammensetzung zur Verwendung gemäß Anspruch 8, wobei das Taxan Paclitaxel ist.

10. Zusammensetzung zur Verwendung gemäß einem der Ansprüche 1-9, wobei das Colchicin- oder Thiocolchicindimer, der Anti-VEGF-Antikörper und das Taxan zeitgleich bzw. simultan verabreicht werden.

11. Zusammensetzung zur Verwendung gemäß einem der Ansprüche 1-10, wobei das Verfahren intravenöse Verabreicherung von mindestens einem aus Colchicin oder Thiocolchicindimer, dem Anti-VEGF-Antikörper und dem Taxan umfasst.

12. Zusammensetzung zur Verwendung gemäß einem der Ansprüche 1-11, wobei der Anti-VEGF-Antikörper Bevacizumab ist.

13. Zusammensetzung zur Verwendung gemäß einem der Ansprüche 1-12, wobei die proliferative Erkrankung Krebs, ein vaskulärer Tumor, oder irgendeiner aus Brustkrebs, Dickdarmkrebs oder Prostatakrebs, oder ein solider Tumor ist.

14. Zusammensetzung zur Verwendung gemäß einem der Ansprüche 1-13, wobei das Individuum ein Mensch ist.

## Revendications

1. Composition comprenant un dimère de colchicine ou de thiocolchicine pour l'utilisation dans une méthode de traitement d'une maladie proliférative chez un individu, dans laquelle la méthode comprend en outre l'administration d'un anticorps anti-VEGF et d'un taxane.

2. Composition pour l'utilisation selon la revendication 1, dans laquelle le dimère de colchicine ou de thiocolchicine est IDN-5404.

3. Composition pour l'utilisation selon la revendication 1 ou 2, dans laquelle le dimère de colchicine ou de thiocolchicine est sous la forme de nanoparticules.

4. Composition pour l'utilisation selon la revendication 3, dans laquelle les nanoparticules comprennent le dimère de colchicine ou de thiocolchicine et une albumine, de préférence le dimère de colchicine ou de thiocolchicine revêtu par l'albumine.

5. Composition pour l'utilisation selon la revendication 4, dans laquelle le diamètre moyen des nanoparticules dans la composition n'est pas supérieur à 200 nm.

6. Composition pour l'utilisation selon l'une quelconque des revendications 1 à 5, dans laquelle le taxane est sous la forme de nanoparticules.

7. Composition pour l'utilisation selon la revendication 6, dans laquelle la nanoparticule de taxane comprend le taxane et une albumine, de préférence le taxane revêtu par l'albumine.

8. Composition pour l'utilisation selon la revendication 6 ou 7, dans laquelle le diamètre moyen des nanoparticules de taxane n'est pas supérieur à 200 nm.

9. Composition pour l'utilisation selon la revendication 8, dans laquelle le taxane est le paclitaxel.

10. Composition pour l'utilisation selon l'une quelconque des revendications 1 à 9, dans laquelle le dimère de colchicine ou de thiocolchicine, l'anticorps anti-VEGF et le taxane sont administrés conjointement ou simultanément.

11. Composition pour l'utilisation selon l'une quelconque des revendications 1 à 10, dans laquelle la méthode comprend l'administration intraveineuse d'au moins un parmi le dimère de colchicine ou de thiocolchicine, l'anticorps anti-VEGF et le taxane.

12. Composition pour l'utilisation selon l'une quelconque des revendications 1 à 11, dans laquelle l'anticorps anti-VEGF est le bévacizumab.

13. Composition destinée à être utilisée selon l'une quelconque des revendications 1 à 12, dans laquelle la maladie proliférante est un cancer, de préférence une tumeur vasculaire, ou l'un parmi le cancer du sein, le cancer du côlon, ou le cancer de la prostate, ou une tumeur solide.

14. Composition pour l'utilisation selon l'une quelconque des revendications 1 à 13, dans laquelle l'individu est un être humain.
